# EUROPEAN PATENT APPLICATION

(11) **EP 4 414 381 A1**
(43) Date of publication of application: **14.08.2024**
(21) Application number: 22889269.1
(22) Date of filing: 01.11.2022
(51) Int. Cl.: C07K 14/54, C07K 16/18, C07K 19/00, C12N 15/26, A61K 39/395

(54) **IL-10 MONOMER FUSION PROTEIN AND USE THEREOF**

(30) Priority: 02.11.2021 CN 202111286063
(71) Applicant: Guangdong Fapon Biopharma Inc., Dongguan, Guangdong 523808 (CN)
(72) Inventor: LU, Di, Dongguan, Guangdong 523808 (CN); HUO, Yongting, Dongguan, Guangdong 523808 (CN); ZHANG, Yibo, Dongguan, Guangdong 523808 (CN); YAN, Jiaqing, Dongguan, Guangdong 523808 (CN)
(74) Representative: Studio Torta S.p.A.
(86) International application number: PCT/CN2022/128998
(87) International publication number: WO 2023/078245

(57) **Abstract**

Provided are an IL-10 monomer fusion protein and use thereof, which relate to the field of biomedicine. The fusion protein includes an antibody and an IL-10 monomer. The fusion protein of the present disclosure has a relatively strong targeting property, so that the safety is remarkably improved. Moreover, the protein may effectively treat or prevent various tumors or inflammatory diseases.

## Description

### Cross-Reference to Related Application

The present application claims priority to Chinese patent application No. 202111286063.4, filed on November 2, 2021 and entitled "IL10 Monomer Fusion Protein and Application thereof", the disclosure of which is hereby incorporated by reference in its entirety.

### Technical Field

The present disclosure relates to the field of biomedicine, specifically, relates to a fusion protein and an application thereof, and more specifically, relates to a fusion protein, a nucleic acid, an expression vector, a recombinant cell, a use of the fusion protein or the nucleic acid or the expression vector or the recombinant cell in preparation of a drug, and a pharmaceutical composition.

### Background

Interleukin-10 (IL-10 or IL10), also known as a human cytokine synthesis inhibitory factor (CSIF), is an anti-inflammatory cytokine, and belongs to homologous dimer secretions. At present, there are safety problems in the IL-10 protein clinically, a small amount of naturally dimerized IL-10 molecules bind to its receptor IL-10Rα in high affinity, and further bind to IL-10Rβ, to form a hexamer complex and activate a downstream signal, thus a biological functional response is caused.

At present, clinical IL-10 drug molecules lack the targeting property, so the drug molecules are prone to off-target and produce side effects; and most of IL-10 antibody fusion proteins under development are designed to utilize the antibody targeting to introduce IL-10 into the local environment and exert its effects. These fusion protein molecules often have a normal IL-10 dimer structure, but due to the high affinity binding between the IL-10 dimer and IL-10Rα, the targeting of the antibody terminus may be affected, thereby the design failure is caused. In addition, if the activity of the antibody terminus requires a relatively large dose, the side effects of the IL-10 terminus are still a thorny problem.

Therefore, it is still necessary to actively explore IL-10 drugs with strong targeting and small side effects, and this research direction is of great significance for prevention or treatment of tumors or inflammatory diseases.

### Summary

The present application is made on the basis of discovery and understanding of the following problems by the inventor.

At present, clinical IL-10 drugs have the problems of lack of targeting property and easy off-target of drug molecules, not only the efficacy of the drugs is not easy to exert, but also the side effects are easily produced. The inventor, by exploring a linkage mode between an IL-10 monomer molecule and an antibody molecule, prepared a fusion protein containing the IL-10 monomer molecule and the antibody molecule. The IL-10 molecule in the fusion protein is in monomer form before an antibody binds to a target cell, it may not bind to its receptor, and may not interfere with the binding of the antibody molecule to an antigen on the target cell. After the antibody binds to the antigen, a large number of IL-10 monomer molecules are aggregated, so that the two IL-10 monomers that are close to each other act as a dimer and bind to the IL-10 receptor, thereby a normal biological response is caused, the targeting property and safety of the IL-10 drugs are significantly improved, and the efficacy is fully exerted.

In the first aspect of the present disclosure, the present disclosure provides a fusion protein. According to an embodiment of the present disclosure, the fusion protein includes an antibody and an IL-10 monomer, herein the antibody includes two identical light chains and two identical heavy chains, one IL-10 monomer is linked to the heavy chain or the light chain of the antibody, and an N-terminus of the IL-10 monomer is not linked to a C-terminus of the heavy chain of the antibody. The natural IL-10 molecule is in dimer form, but the IL-10 molecule in the fusion protein according to the embodiment of the present disclosure is in monomer form before the antibody binds to the target cell, it may not bind to its receptor, and may not interfere with the binding of the antibody molecule to the target cell. After the antibody binds to the target cell, a large number of IL-10 monomer molecules are aggregated, so that the two IL-10 monomers that are close to each other act as a dimer and bind to the IL-10 receptor, thereby the normal biological response is caused, and since the IL-10 monomer is difficult to trigger a biological function, the IL-10 monomer fusion protein does not produce limitations on administration doses as those of the natural dimer IL-10 molecules or its antibody fusion proteins, so that the administration dose of the IL-10 monomer fusion protein is significantly improved. The fusion protein may be used in combination with more antibody molecules, so that the targeting property and safety of the fusion protein are significantly improved, the efficacy is fully exerted, and it may treat or prevent various tumors or inflammatory diseases.

According to an embodiment of the present disclosure, the above fusion protein may further include at least one of the following additional technical features.

According to an embodiment of the present disclosure, each light chain of the antibody includes a VL region and a CL region, and each heavy chain of the antibody includes a VH region, a CH1 region, a CH2 region, and a CH3 region; a N-terminus of the IL-10 monomer is linked to a C-terminus of the light chain of the antibody; or a C-terminus of the IL-10 monomer is linked to a N-terminus of the light chain of the antibody; or the N-terminus of the IL-10 monomer is linked to a C-terminus of the VL region of the light chain, and the C-terminus of the IL-10 monomer is linked to a N-terminus of the CL region of the light chain; or the C-terminus of the IL-10 monomer is linked to a N-terminus of the heavy chain of the antibody; or the N-terminus of the IL-10 monomer is linked to a C-terminus of the VH region of the heavy chain, and the C-terminus of the IL-10 monomer is linked to a N-terminus of the CH1 region of the heavy chain; or the N-terminus of the IL-10 monomer is linked to a C-terminus of the CH2 region of the heavy chain, and the C-terminus of the IL-10 monomer is linked to a N-terminus of the CH3 region of the heavy chain.

According to an embodiment of the present disclosure, the IL-10 monomer includes a natural IL-10 monomer.

According to an embodiment of the present disclosure, the IL-10 monomer is a modified natural IL-10 monomer, and the modification includes at least one of (i) and (ii): (i) the first two amino acids at the N-terminus of the natural IL-10 monomer are deleted; and (ii) a first linker peptide is inserted between the 116-th amino acid and the 117-th amino acid, and the first linker peptide is a flexible linker peptide.

After the first 2 amino acids at the N-terminus of the natural IL-10 monomer are deleted, the stability of the IL-10 monomer is improved. At the same time, after the first linker peptide is inserted between the 116-th amino acid and the 117-th amino acid of the IL-10 monomer, the IL-10 monomer molecule in the fusion protein is blocked, and becomes a closed-ring IL-10 monomer molecule (two IL10 chains of a natural IL10 dimer are formed by interlacing together, and after being modified by inserting a linker, the IL10 monomer may independently form a domain, namely the closed-ring monomer molecule), two IL-10 monomers in the same fusion protein may not produce interference such as mutual linkage.

According to an embodiment of the present disclosure, the amino acid sequence of the first linker peptide is shown in SEQ ID NO: 21.
GGGSGG (SEQ ID NO: 21).

According to an embodiment of the present disclosure, the IL-10 monomer has an amino acid sequence as shown in SEQ ID NO: 2.

GQGTQSENSCTHFPGNLPNMLRDLRDAFSRVKTFFQMKDQLDNLLLKESLLEDFKGYLG

CQALSEMIQFYLEEVMPQAENQDPDIKAHVNSLGENLKTLRLRLRRCHRFLPCENGGGSGGKS
KAVEQVKNAFNKLQEKGIYKAMSEFDIFINYIEAYMTMKIRN (SEQ ID NO: 2).

After the IL-10 monomer is linked to the heavy chain or the light chain of the antibody with the second linker peptide or/and the third linker peptide, an antibody portion in the fusion protein binds to the corresponding target cell, and the two mutually close IL-10 monomers of the fusion proteins are more likely to form the dimer to function and bind to the IL-10 receptor.

According to an embodiment of the present disclosure, the second linker peptide or/and the third linker peptide may be a flexible linker peptide or a rigid linker peptide.

According to an embodiment of the present disclosure, the second linker peptide has an amino acid sequence as shown in SEQ ID NO: 12, and the third linker peptide has an amino acid sequence as shown in SEQ ID NO: 22.
GSGSGSGS (SEQ ID NO: 12).
GGGGSGGGGSGGGGSGGGGS (SEQ ID NO: 22).

According to an embodiment of the present disclosure, the IL-10 monomer is linked to a heavy chain terminus or a light chain terminus of the antibody by the second linker peptide.

According to an embodiment of the present disclosure, a N-terminus of the second linker peptide is linked to the C-terminus of the light chain of the antibody, and a C-terminus of the second linker peptide is linked to the N-terminus of the IL-10 monomer; or the N-terminus of the second linker peptide is linked to the C-terminus of the IL-10 monomer, and the C-terminus of the second linker peptide is linked to the N-terminus of the light chain of the antibody; or the N-terminus of the second linker peptide is linked to the C-terminus of the IL-10 monomer, and the C-terminus of the second linker peptide is linked to the N-terminus of the heavy chain of the antibody.

According to an embodiment of the present disclosure, the IL-10 monomer is linked to the heavy chain terminus or the light chain terminus of the antibody by the third linker peptide.

According to an embodiment of the present disclosure, a N-terminus of the third linker peptide is linked to the C-terminus of the light chain of the antibody, and a C-terminus of the third linker peptide is linked to the N-terminus of the IL-10 monomer; or the N-terminus of the third linker peptide is linked to the C-terminus of the IL-10 monomer, and the C-terminus of the third linker peptide is linked to the N-terminus of the light chain of the antibody; or the N-terminus of the third linker peptide is linked to the C-terminus of the IL-10 monomer, and the C-terminus of the third linker peptide is linked to the N-terminus of the heavy chain of the antibody.

According to an embodiment of the present disclosure, the N-terminus of the third linker peptide is linked to the C-terminus of the VL region of the light chain, and the C-terminus of the third linker peptide is linked to the N-terminus of the IL-10 monomer; or the N-terminus of the third linker peptide is linked to the C-terminus of the IL-10 monomer, and the C-terminus of the third linker peptide is linked to the N-terminus of the CL region of the light chain; or the N-terminus of the third linker peptide is linked to the C-terminus of the VH region of the heavy chain, and the C-terminus of the third linker peptide is linked to the N-terminus of the IL-10 monomer; or the N-terminus of the third linker peptide is linked to the C-terminus of the IL-10 monomer, and the C-terminus of the third linker peptide is linked to the N-terminus of the CH1 region of the heavy chain; or the N-terminus of the third linker peptide is linked to the C-terminus of the CH2 region of the heavy chain, and the C-terminus of the third linker peptide is linked to the N-terminus of the IL-10 monomer; or the N-terminus of the third linker peptide is linked to the C-terminus of the IL-10 monomer, and the C-terminus of the third linker peptide is linked to the N-terminus of the CH3 region of the heavy chain.

According to an embodiment of the present disclosure, the N-terminus of the IL-10 monomer is linked to the C-terminus of the VL region of the light chain of the antibody by the second linker peptide, and the C-terminus of the IL-10 monomer is linked to the N-terminus of the CL region of the light chain of the antibody by the second linker peptide; or the N-terminus of the IL-10 monomer is linked to the C-terminus of the VH region of the heavy chain of the antibody by the second linker peptide, and the C-terminus of the IL-10 monomer is linked to the N-terminus of the CH1 region of the heavy chain of the antibody by the second linker peptide; or the N-terminus of the IL-10 monomer is linked to the C-terminus of the CH2 region of the heavy chain of the antibody by the second linker peptide, and the C-terminus of the IL-10 monomer is linked to the N-terminus of the CH3 region of the heavy chain of the antibody by the second linker peptide.

According to an embodiment of the present disclosure, the antibody is selected from an immune cell antibody and a tumor cell antibody.

According to an embodiment of the present disclosure, the antibody is an anti-PD-1 antibody, an anti-PD-L1 antibody, an anti-Her2 antibody, an anti-CCR8 antibody, an anti-VEGFR2 antibody, an anti-claudin18.2 antibody, an anti-CD39 antibody, an anti-SMA4D antibody, an anti-GUCY2C antibody, an anti-CTLA-4 antibody, an anti-TIM3 antibody, an anti-TIGIT antibody, an anti-CD47 antibody, or an anti-TNF α antibody.

According to an embodiment of the present disclosure, the fusion protein has amino acid sequences as shown in SEQ ID NO: 3 and SEQ ID NO: 9; or the fusion protein has amino acid sequences as shown in SEQ ID NO: 3 and SEQ ID NO: 5; or the fusion protein has amino acid sequences as shown in SEQ ID NO: 4 and SEQ ID NO: 6; or the fusion protein has amino acid sequences as shown in SEQ ID NO: 7 and SEQ ID NO: 6; or the fusion protein has amino acid sequences as shown in SEQ ID NO: 3 and SEQ ID NO: 8; or the fusion protein has amino acid sequences as shown in SEQ ID NO: 10 and SEQ ID NO: 6.

QVQLKESGPGLVQPSQTLSLTCTVSGFSLTSYNVHWVRQPPGKGLEWMGGMRYNEDTS
YNSALKSRLSISRDTSKNQVFLKMNSLQTDDTGTYYCTRDAVYGGYGGWFAYWGQGTLVTVS
SASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEAAGGPSVFLF
PPKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFSCSVMHEAL
HNHYTQKSLSLSPGK (SEQ ID NO: 3).

DTVLTQSPALAVSLGQRVTISCKASETVSSSMYSYIHWYQQKPGQQPKLLIYRASNLESGV
PARFSGSGSGTDFTLTIDPVEADDVATYFCQQSWNPWTFGGGTKLELKRTVAAPSVFIFPPSDE
QLKSGTASVVCLLNNFYPREAKVQWKVDNALQSGNSQESVTEQDSKDSTYSLSSTLTLSKADY
EKHKVYACEVTHQGLSSPVTKSFNRGECGGGGSGGGGSGGGGSGGGGSGGQGTQSENSC
THFPGNLPNMLRDLRDAFSRVKTFFQMKDQLDNLLLKESLLEDFKGYLGCQALSEMIQFYLEEV
MPQAENQDPDIKAHVNSLGENLKTLRLRLRRCHRFLPCENGGGSGGKSKAVEQVKNAFNKLQ
EKGIYKAMSEFDIFINYIEAYMTMKIRN (SEQ ID NO: 9).

DTVLTQSPALAVSLGQRVTISCKASETVSSSMYSYIHWYQQKPGQQPKLLIYRASNLESGV
PARFSGSGSGTDFTLTIDPVEADDVATYFCQQSWNPWTFGGGTKLELKRGSGSGSGSGQGTQ
SENSCTHFPGNLPNMLRDLRDAFSRVKTFFQMKDQLDNLLLKESLLEDFKGYLGCQALSEMIQ
FYLEEVMPQAENQDPDIKAHVNSLGENLKTLRLRLRRCHRFLPCENGGGSGGKSKAVEQVKN
AFNKLQEKGIYKAMSEFDIFINYIEAYMTMKIRNGSGSGSGSTVAAPSVFIFPPSDEQLKSGTASV
VCLLNNFYPREAKVQWKVDNALQSGNSQESVTEQDSKDSTYSLSSTLTLSKADYEKHKVYACE
VTHQGLSSPVTKSFNRGEC (SEQ ID NO: 5).

QVQLKESGPGLVQPSQTLSLTCTVSGFSLTSYNVHWVRQPPGKGLEWMGGMRYNEDTS
YNSALKSRLSISRDTSKNQVFLKMNSLQTDDTGTYYCTRDAVYGGYGGWFAYWGQGTLVTVS
SGSGSGSGSGQGTQSENSCTHFPGNLPNMLRDLRDAFSRVKTFFQMKDQLDNLLLKESLLED
FKGYLGCQALSEMIQFYLEEVMPQAENQDPDIKAHVNSLGENLKTLRLRLRRCHRFLPCENGG
GSGGKSKAVEQVKNAFNKLQEKGIYKAMSEFDIFINYIEAYMTMKIRNGSGSGSGSASTKGPSV
FPLAPSSKSTSGGTAALGCLVKDYFPEPVTVSWNSGALTSGVHTFPAVLQSSGLYSLSSVVTVP
SSSLGTQTYICNVNHKPSNTKVDKRVEPKSCDKTHTCPPCPAPEAAGGPSVFLFPPKPKDTLMI

SRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHYTQKSLS
LSPGK (SEQ ID NO: 4).

DTVLTQSPALAVSLGQRVTISCKASETVSSSMYSYIHWYQQKPGQQPKLLIYRASNLESGV
PARFSGSGSGTDFTLTIDPVEADDVATYFCQQSWNPWTFGGGTKLELKRTVAAPSVFIFPPSDE
QLKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO: 6).

QVQLKESGPGLVQPSQTLSLTCTVSGFSLTSYNVHWVRQPPGKGLEWMGGMRYNEDTS
YNSALKSRLSISRDTSKNQVFLKMNSLQTDDTGTYYCTRDAVYGGYGGWFAYWGQGTLVTVS
SASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEAAGGPSVFLF
PPKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGSGSGSGSGQGTQSENSCTHFPGNLPNM
LRDLRDAFSRVKTFFQMKDQLDNLLLKESLLEDFKGYLGCQALSEMIQFYLEEVMPQAENQDP
DIKAHVNSLGENLKTLRLRLRRCHRFLPCENGGGSGGKSKAVEQVKNAFNKLQEKGIYKAMSE
FDIFINYIEAYMTMKIRNGSGSGSGSGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSL
SLSPGK (SEQ ID NO: 7).

GQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENGGGSGGKS
KAVEQVKNAFNKLQEKGIYKAMSEFDIFINYIEAYMTMKIRNGGGGSGGGGSGGGGSGGGGS
GDTVLTQSPALAVSLGQRVTISCKASETVSSSMYSYIHWYQQKPGQQPKLLIYRASNLESGVPA
RFSGSGSGTDFTLTIDPVEADDVATYFCQQSWNPWTFGGGTKLELKRTVAAPSVFIFPPSDEQL
KSGTASVVCLLNNFYPREAKVQWKVDNALQSGNSQESVTEQDSKDSTYSLSSTLTLSKADYEK
HKVYACEVTHQGLSSPVTKSFNRGEC (SEQ ID NO: 8).

GQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENGGGSGGKS
KAVEQVKNAFNKLQEKGIYKAMSEFDIFINYIEAYMTMKIRNGGGGSGGGGSGGGGSGGGGS
GQVQLKESGPGLVQPSQTLSLTCTVSGFSLTSYNVHWVRQPPGKGLEWMGGMRYNEDTSYN
SALKSRLSISRDTSKNQVFLKMNSLQTDDTGTYYCTRDAVYGGYGGWFAYWGQGTLVTVSSA
STKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKRVEPKSCDKTHTCPPCPAPEAAGGPSVFLFPP

KPKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLVKGF
YPSDIAVEWESNGQPENNYKTTPPVLDSDGSFFLYSKLTVDKSRWQQGNVFSCSVMHEALHN
HYTQKSLSLSPGK (SEQ ID NO: 10).

In the second aspect of the present disclosure, the present disclosure provides a nucleic acid. According to an embodiment of the present disclosure, the nucleic acid encodes the fusion protein as described in the first aspect. The IL-10 molecule in the fusion protein encoded by the nucleic acid according to the embodiment of the present disclosure is in monomer form before the antibody portion binds to the target cell, it may not bind to its receptor, and may not interfere with the binding of the antibody molecule to the target cell; after the antibody binds to the target cell, a large number of IL-10 monomer molecules are aggregated, so that the two IL-10 monomers that are close to each other act as a dimer and bind to the IL-10 receptor, thereby the normal biological response is caused; and since the IL-10 monomer is difficult to trigger the biological function, the IL-10 monomer fusion protein does not produce limitations on administration doses as those of the natural dimer IL-10 molecules or its antibody fusion proteins, so that the administration dose of the IL-10 monomer fusion protein is significantly improved. The fusion protein may be used in combination with more antibody molecules, so that the targeting property and safety of the fusion protein are significantly improved, the efficacy is fully exerted, and it may treat or prevent various tumors or inflammatory diseases.

According to an embodiment of the present disclosure, the nucleic acid molecule is deoxyribonucleic acid (DNA) or ribonucleic acid (RNA).

In the third aspect of the present disclosure, the present disclosure provides an expression vector. According to an embodiment of the present disclosure, the expression vector contains the nucleic acid as described in the second aspect. After the expression vector according to the embodiment of the present disclosure is introduced into an appropriate receptor cell, the expression of the fusion protein mentioned above may be effectively achieved under the mediation of a regulatory system. The IL-10 molecule in the fusion protein obtained is in monomer form before the antibody portion binds to the target cell, it may not bind to its receptor, and may not interfere with the binding of the antibody molecule to the target cell; after the antibody binds to the target cell, a large number of IL-10 monomer molecules are aggregated, so that the two IL-10 monomers that are close to each other act as a dimer and bind to the IL-10 receptor, thereby the normal biological response is caused; and since the IL-10 monomer is difficult to trigger the biological function, the IL-10 monomer fusion protein does not produce limitations on administration doses as those of the natural dimer IL-10 molecules or its antibody fusion proteins, so that the administration dose of the IL-10 monomer fusion protein is significantly improved. The fusion protein may be used in combination with more antibody molecules, so that the targeting property and safety of the fusion protein are significantly improved, the efficacy is fully exerted, and it may treat or prevent various tumors or inflammatory diseases.

In the fourth aspect of the present disclosure, the present disclosure provides a recombinant cell. According to an embodiment of the present disclosure, the recombinant cell carries the nucleic acid as described in the second aspect, and the expression vector as described in the third aspect, or expresses the fusion protein as described in the first aspect. The recombinant cell according to the embodiment of the present disclosure may be used for the expression and large-scale acquisition of the fusion protein mentioned above. The IL-10 molecule in the fusion protein obtained is in monomer form before the antibody portion binds to the target cell, it may not bind to its receptor, and may not interfere with the binding of the antibody molecule to the target cell; after the antibody binds to the target cell, a large number of IL-10 monomer molecules are aggregated, so that the two IL-10 monomers that are close to each other act as a dimer and bind to the IL-10 receptor, thereby the normal biological response is caused; and since the IL-10 monomer is difficult to trigger the biological function, the IL-10 monomer fusion protein does not produce limitations on administration doses as those of the natural dimer IL-10 molecules or its antibody fusion proteins, so that the administration dose of the IL-10 monomer fusion protein is significantly improved. The fusion protein may be used in combination with more antibody molecules, so that the targeting property and safety of the fusion protein are significantly improved, the efficacy is fully exerted, and it may treat or prevent various tumors or inflammatory diseases.

In the fifth aspect of the present disclosure, the present disclosure provides a use of the fusion protein as described in the first aspect, the nucleic acid as described in the second aspect, the expression vector as described in the third aspect, or the recombinant cell as described in the fourth aspect in preparation of a drug. According to an embodiment of the present disclosure, the drug is used to treat or prevent tumors or inflammatory diseases. According to a specific embodiment of the present disclosure, the drug may effectively treat or alleviate the tumors and inflammatory diseases, and has the relatively high safety.

In the sixth aspect of the present disclosure, the present disclosure provides a pharmaceutical composition. According to an embodiment of the present disclosure, it contains the fusion protein as described in the first aspect, the nucleic acid as described in the second aspect, the expression vector as described in the third aspect, or the recombinant cell as described in the fourth aspect. The pharmaceutical composition according to the specific embodiment of the present disclosure may effectively treat or alleviate the tumors and inflammatory diseases, and has the relatively high safety.

According to an embodiment of the present disclosure, the above pharmaceutical composition may further include at least one of the following additional technical features (i) and (ii):
(i) According to an embodiment of the present disclosure, the pharmaceutical composition is used to treat or prevent tumors or inflammatory diseases.
(ii) According to an embodiment of the present disclosure, the pharmaceutical composition may include: a pharmaceutically acceptable excipient, and the pharmaceutically acceptable excipient includes at least one of a stabilizer, a wetting agent, an emulsifier, an adhesive, and an isotonic agent; and the pharmaceutical composition is in the form of at least one of tablet, granule, powder, capsule, solution, suspension, and lyophilized preparation.

In the seventh aspect of the present disclosure, the present disclosure provides a method for diagnosing, treating, preventing, or alleviating diseases, symptoms, or conditions related to tumors or inflammatory diseases in a subject, and it includes administering a therapeutically effective amount of the aforementioned fusion protein and/or the aforementioned pharmaceutical composition to the above subject.

The additional aspects and advantages of the present disclosure are partially given in the following description, some of which may become apparent from the following description, or may be understood by the practice of the present disclosure.

### Brief Description of the Drawings

The above and/or additional aspects and advantages of the present disclosure, combined with the description of embodiments in drawings, may become apparent and easy to understand, herein:

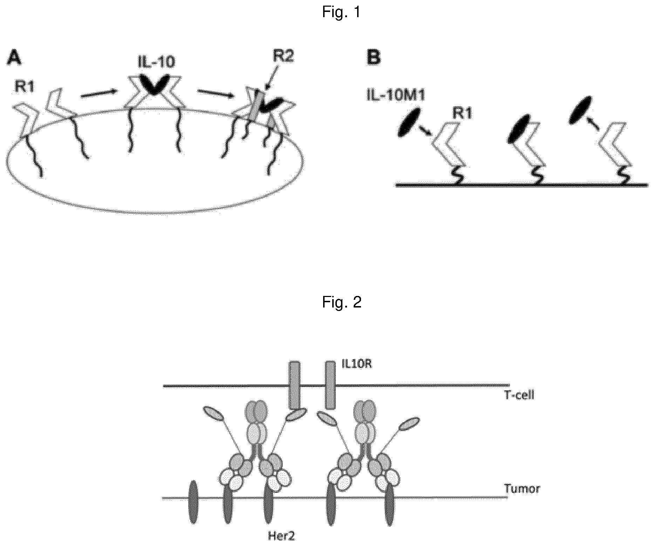

Fig. 1 is a schematic diagram of a binding mode of a natural IL-10 molecule and an IL-10 monomer to an IL-10 receptor according to an embodiment of the present disclosure, herein A represents a schematic diagram of a binding mode of the natural IL-10 molecule to the IL-10 receptor, R1 represents the IL-10 receptor (IL-10Rα), and R2 represents the IL-10 receptor (IL-10Rβ); and B represents a schematic diagram of a binding mode of the IL-10 monomer to the IL-10 receptor, and R1 represents the IL-10 receptor (IL-10Rα), and IL-10M1 represents the IL-10 monomer;
Fig. 2 is a schematic diagram of binding of the antibody to an antigen on a target cell and binding of the IL-10 monomer to the IL-10 receptor in the fusion protein according to an embodiment of the present disclosure. The IL-10 monomer has the relatively weak affinity and poor targeting effect, so the antibody terminus may play a normal targeting effect. After being targeted to the tumor surface, the local enrichment of the antibody may aggregate the IL-10 monomers together and form a dimer IL-10(M)*2 to play an effect, herein IL-10R represents an IL10RαRβ complex, Her2 represents a human epidermal growth factor receptor, which is an example of an antibody targeting antigen, T-cell represents a T cell, and Tumor represents a tumor;

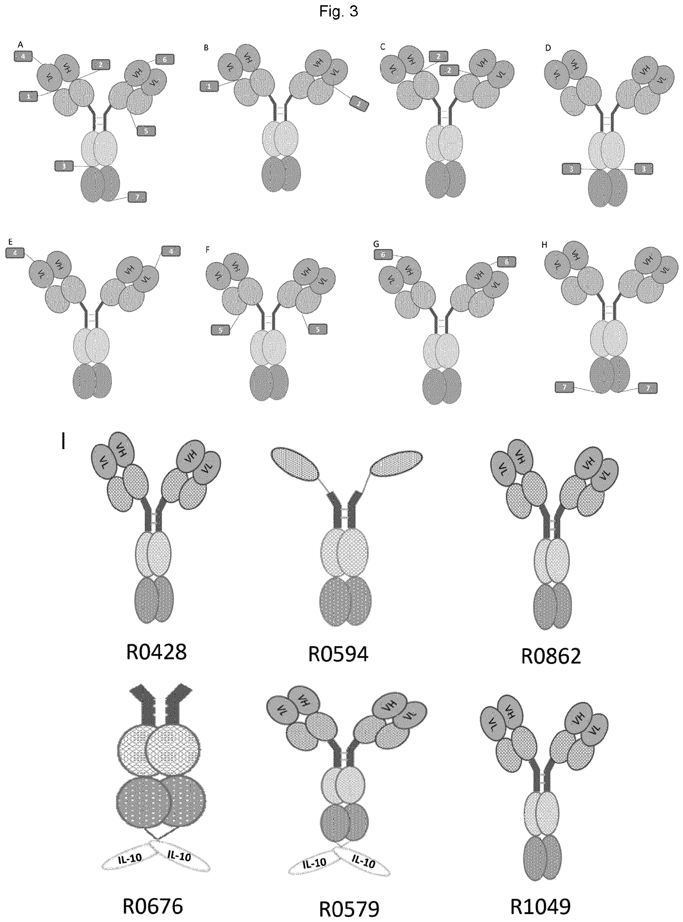

Fig. 3 is a structure schematic diagram of a position of the IL-10 monomer inserted into the antibody molecule according to an embodiment of the present disclosure, herein A represents an insertable position of the IL-10 monomer on the antibody (Positions 1-7), and B represents a structure schematic diagram of inserting one IL-10 monomer between VL and CL (namely Position 1) of each light chain of the antibody, and being linked by linker2, and the fusion protein molecule obtained is named as R0987; C represents a structure schematic diagram of inserting one IL-10 monomer between VH and CH1 (namely Position 2) of a heavy chain of the antibody, and being linked by linker2, and the fusion protein molecule obtained is named as R0988; D represents a structure schematic diagram of inserting one IL-10 monomer between CH2 and CH3 (namely Position 3) of the heavy chain of the antibody, and being linked by linker2, and the fusion protein molecule obtained is named as R0989; E represents a structure schematic diagram of inserting one IL-10 monomer at a N-terminus (namely Position 4) of the light chain of the antibody, and being linked by linker2, and the fusion protein molecule obtained is named as R0990; F represents a structure schematic diagram of inserting one IL-10 monomer at a C-terminus (namely Position 5) of the light chain of the antibody, and being linked by linker3, and the fusion protein molecule obtained is named as R0991; G represents a structure schematic diagram of inserting one IL-10 monomer at a N-terminus (namely Position 6 of the heavy chain of the antibody, and being linked by linkers, and the fusion protein molecule obtained is named as R0992; H represents a structure schematic diagram of inserting one IL-10 monomer at a C-terminus (namely Position 7) of the heavy chain of the antibody, and being linked by linker3, and the fusion protein molecule obtained is named as R0993; and I represents a structure diagram of control molecules (R0428, R0594, R0862, R0676, R0579, and R1049) in Embodiments 3-5 of the present disclosure;

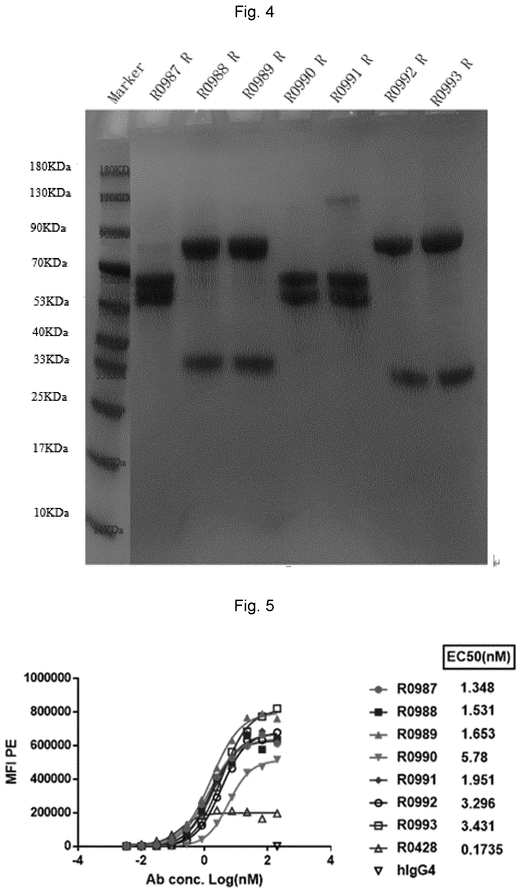

Fig. 4 is an SDS-PAGE detection result diagram of the fusion proteins R0987, R0988, R0989, R0990, R0991, R0992, and R0993 according to an embodiment of the present disclosure;
Fig. 5 is a result diagram of detecting antibody terminus binding activity of the fusion protein with a flow cytometry according to an embodiment of the present disclosure, herein the horizontal ordinate (Ab conc. Log (nM)) represents an antibody concentration (nM), and the vertical ordinate (MFI PE) represents an average fluorescence intensity;

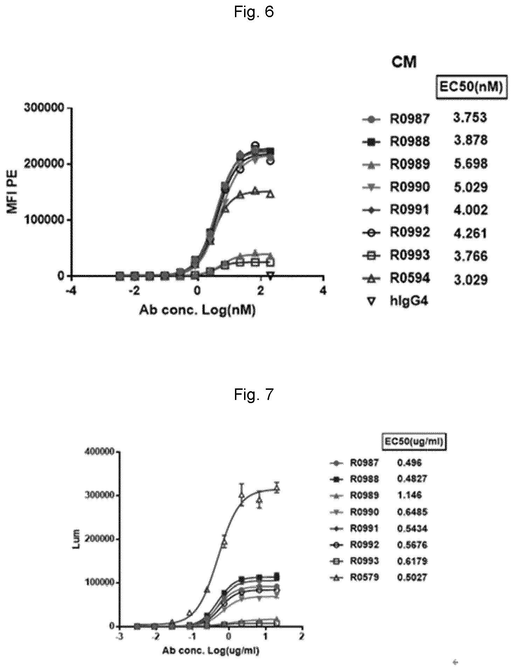

Fig. 6 is a result diagram of detecting IL-10 monomer terminus binding activity of the fusion protein with the flow cytometry according to an embodiment of the present disclosure, herein the horizontal ordinate (Ab conc. Log (nM)) represents the antibody concentration (nM), and the vertical ordinate (MFI PE) represents the average fluorescence intensity;
Fig. 7 is a result diagram of detecting IL-10 monomer terminus binding activity of the fusion protein with a reporter gene method according to an embodiment of the present disclosure, herein the horizontal ordinate (Ab conc. Log)(µg/mL) represents the antibody concentration (µg/mL), and the vertical ordinate (Lum) represents a Luminescence fluorescence intensity;

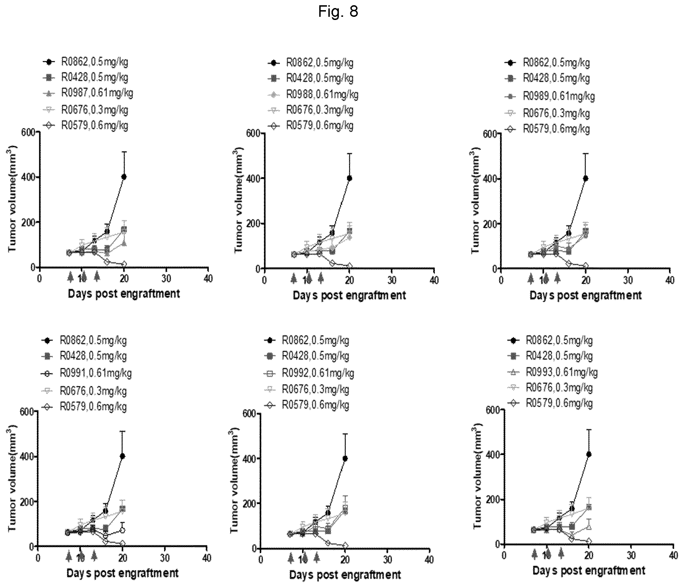

Fig. 8 is a statistical analysis result diagram of a tumor volume in a MC38 tumor model mouse treated with the fusion protein according to an embodiment of the present disclosure, herein the horizontal ordinate (Days post engraftment) represents the number of days after mouse engraftment, and the vertical ordinate (Tumor volume) represents the tumor volume; and

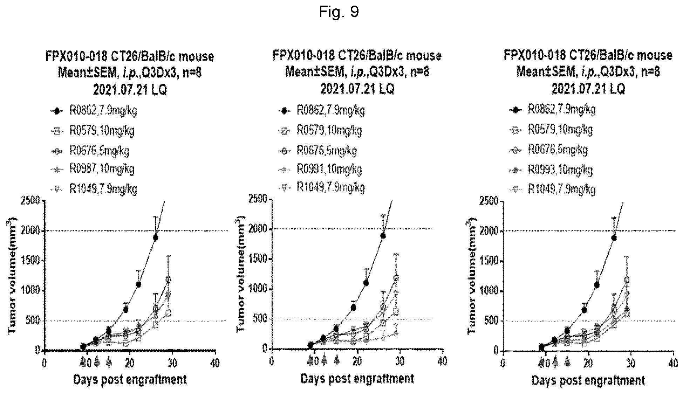

Fig. 9 is a statistical analysis result diagram of a tumor volume in a CT26 tumor model mouse treated with the fusion protein according to an embodiment of the present disclosure, herein the horizontal ordinate (Days post engraftment) represents the number of days after mouse engraftment, and the vertical ordinate (Tumor volume) represents the tumor volume.

### Detailed Description of the Embodiments

Embodiments of the present disclosure are described in detail below, and examples of the embodiments are shown in drawings. The embodiments described below with reference to the drawings are exemplary and intended to explain the present disclosure, but may not be understood as limitations on the present disclosure.

In addition, terms "first" and "second" are only used for a descriptive purpose, and may not be understood as indicating or implying relative importance or implying the quantity of technical features indicated. Therefore, the features limited to the "first" and "second" may explicitly or implicitly include at least one of these features. In the description of the present disclosure, "plurality" means at least two, such as two and three, unless otherwise specified.

In this article, the term "antibody" refers to an immunoglobulin molecule that may specifically bind to an antigen, including two light chains with the lighter molecular weight and two heavy chains with the heavier molecular weight, the heavy chain (H chain) and the light chain (L chain) are linked by a disulfide bond to form a tetrapeptide chain molecule. Herein, the amino acid sequence at an amino end (N-terminus) of the peptide chain is significantly changed, known as a variable region (V region), while a carboxyl end (C-terminus) is relatively stable, and the change is small, known as a constant region (C region). The V region of the L chain and the H chain are called as VL and VH respectively. The C region of the L chain is called as CL, and the C region of the H chain contains CH1, CH2, and CH3 regions.

The natural IL-10 molecule is a dimer structure (including two natural IL-10 monomers). The binding mode of the natural IL-10 molecule to the IL-10 receptor (IL-1 0R) is shown in Fig. 1-A, and the activity of the IL-10 monomer against IL-10Rα is 10 times weaker than that of the natural IL-10 dimer (specific experimental data refers to a document: J Biol Chem.2000 May 5; 275(18):13552-7.doi:10.1074/jbc.275.18.13552.), and it is almost unable to mediate the further binding to IL-10Rβ. Therefore, it is very difficult to activate a downstream signal to cause a biological functional response. The binding mode of the IL-10 monomer to the IL-10 receptor is shown in Fig. 1-B.

Therefore, in the present disclosure, the IL-10 monomers are linked to different portions (it may be a symmetric or asymmetric structure) of the antibody molecule to construct the fusion protein (IL-10 monomer fusion protein), and the IL-10 monomers are isolated by the spatial hindrance of the own structure of the antibody. When the IL-10 monomer fusion protein is located in a free state, even if the IL-10 monomer binds to IL-10Rα, it may not cause the functional response. Only when the antibody terminus binds to a target cell, neighboring IL-10 monomer-IL-10R complexes may be aggregated by an aggregation effect, and it further binds to IL-10Rβ to form a complex so as to generate the downstream signal and trigger the biological function. The specific binding mode is shown in Fig. 2, this may avoid the interference of the IL-10 molecule on the targeting property of the antibody before drug molecules reach a target site, and clinical side effects may not produced; in addition, since the IL-10 monomer is very difficult to trigger the biological function, the IL-10 monomer fusion protein does not produce limitations on administration doses as those of the natural dimer IL-10 molecules or its antibody fusion proteins, so that the administration dose of the IL-10 monomer fusion protein is significantly improved. At the same time, since the clinical usage doses of different antibodies have a difference, when the usage dose of the IL-10 molecules is relatively low, it may seriously limit selection of antibody targets in the IL-10 monomer fusion protein. Therefore, the fusion protein obtained by the present disclosure provides a wider space for the fused antibody terminus to function, thereby the IL-10 monomer fusion protein may be used in combination with more antibody molecules.

In some implementation schemes, the present disclosure provides a fusion protein, including an antibody and an IL-10 monomer, herein the antibody includes two identical light chains and two identical heavy chains, one IL-10 monomer is linked to the heavy chain or the light chain of the antibody, and an N-terminus of the IL-10 monomer is not linked to a C-terminus of the heavy chain of the antibody. The natural IL-10 molecule is in dimer form, but the IL-10 molecule in the fusion protein according to some specific embodiments of the present disclosure is in monomer form before the antibody binds to the target cell, it may not bind to its receptor, and may not interfere with the binding of the antibody molecule to the target cell. After the antibody binds to the target cell, a large number of IL-10 monomer molecules are aggregated, so that the two IL-10 monomers that are close to each other act as a dimer and bind to the IL-10 receptor, thereby the normal biological response is caused, so that the targeting property and safety of the fusion protein are significantly improved, and the efficacy is fully exerted.

According to some specific implementation schemes of the present disclosure, each light chain of the antibody includes a VL region and a CL region, and each heavy chain of the antibody includes a VH region, a CH1 region, a CH2 region, and a CH3 region; a N-terminus of the IL-10 monomer is linked to a C-terminus of the light chain of the antibody; or a C-terminus of the IL-10 monomer is linked to a N-terminus of the light chain of the antibody; or the N-terminus of the IL-10 monomer is linked to a C-terminus of the VL region of the light chain, and the C-terminus of the IL-10 monomer is linked to a N-terminus of the CL region of the light chain; or the C-terminus of the IL-10 monomer is linked to a N-terminus of the heavy chain of the antibody; or the N-terminus of the IL-10 monomer is linked to a C-terminus of the VH region of the heavy chain, and the C-terminus of the IL-10 monomer is linked to a N-terminus of the CH1 region of the heavy chain; or the N-terminus of the IL-10 monomer is linked to a C-terminus of the CH2 region of the heavy chain, and the C-terminus of the IL-10 monomer is linked to a N-terminus of the CH3 region of the heavy chain.

According to some specific implementation schemes of the present disclosure, the IL-10 monomer includes a natural IL-10 monomer, and the natural IL-10 monomer has an amino acid sequence as shown in SEQ ID NO: 1.

SPGQGTQSENSCTHFPGNLPNMLRDLRDAFSRVKTFFQMKDQLDNLLLKESLLEDFKGYL
GCQALSEMIQFYLEEVMPQAENQDPDIKAHVNSLGENLKTLRLRLRRCHRFLPCENKSKAVEQ
VKNAFNKLQEKGIYKAMSEFDIFINYIEAYMTMKIRN (SEQ ID NO: 1).

According to some specific implementation schemes of the present disclosure, the IL-10 monomer includes a modified natural IL-10 monomer, and the modification includes at least one of (i) and (ii) below: (i) the first 2 amino acids at the N-terminus of the natural IL-10 monomer are removed; and (ii) a first linker peptide is inserted between the 116-th amino acid and the 117-th amino acid, and the first linker peptide is a flexible linker peptide.

After the first 2 amino acids at the N-terminus of the natural IL-10 monomer are removed, the stability of the IL-10 monomer is improved. At the same time, after the first linker peptide is inserted between the 116-th amino acid and the 117-th amino acid of the IL-10 monomer, the IL-10 monomer molecule in the fusion protein becomes a closed-ring IL-10 monomer molecule (two IL10 chains of a natural IL10 dimer are formed by interlacing together, and after being modified by inserting a linker, the IL10 monomer may independently form a domain, namely the closed-ring monomer molecule), two IL-10 monomers in the same fusion protein may not produce interference such as mutual linkage.

According to some specific implementation schemes of the present disclosure, the first linker peptide has an amino acid sequence as shown in in SEQ ID NO: 21.
GGGSGG (SEQ ID NO: 21).

According to some specific implementation schemes of the present disclosure, the IL-10 monomer has an amino acid sequence as shown in SEQ ID NO: 2.

GQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENGGGSGGKS
KAVEQVKNAFNKLQEKGIYKAMSEFDIFINYIEAYMTMKIRN (SEQ ID NO: 2).

According to an embodiment of the present disclosure, the IL-10 monomer is linked to the heavy chain or the light chain of the antibody by a second linker peptide or/and a third linker peptide. After the IL-10 monomer is linked to the heavy chain or the light chain of the antibody by using the second linker peptide or/and the third linker peptide, an antibody portion in the fusion protein binds to the corresponding target cell, and the IL-10 monomers of the 2 mutually close fusion proteins are more likely to form the dimer to act and bind to the IL-10 receptor.

According to an embodiment of the present disclosure, the second linker peptide or/and the third linker peptide are not particularly limited, and may be a flexible linker peptide or a rigid linker peptide.

According to an embodiment of the present disclosure, the second linker peptide has an amino acid sequence as shown in SEQ ID NO: 12, and the third linker peptide has an amino acid sequence as shown in SEQ ID NO: 22.
GSGSGSGS (SEQ ID NO: 12).
GGGGSGGGGSGGGGSGGGGS (SEQ ID NO: 22).

According to an embodiment of the present disclosure, the IL-10 monomer is linked to a heavy chain terminus or a light chain terminus of the antibody by the second linker peptide.

According to an embodiment of the present disclosure, a N-terminus of the second linker peptide is linked to the C-terminus of the light chain of the antibody, and a C-terminus of the second linker peptide is linked to the N-terminus of the IL-10 monomer; or the N-terminus of the second linker peptide is linked to the C-terminus of the IL-10 monomer, and the C-terminus of the second linker peptide is linked to the N-terminus of the light chain of the antibody; or the N-terminus of the second linker peptide is linked to the C-terminus of the IL-10 monomer, and the C-terminus of the second linker peptide is linked to the N-terminus of the heavy chain of the antibody.

According to an embodiment of the present disclosure, the IL-10 monomer is linked to the heavy chain terminus or the light chain terminus of the antibody by the third linker peptide.

According to an embodiment of the present disclosure, a N-terminus of the third linker peptide is linked to the C-terminus of the light chain of the antibody, and a C-terminus of the third linker peptide is linked to the N-terminus of the IL-10 monomer; or the N-terminus of the third linker peptide is linked to the C-terminus of the IL-10 monomer, and the C-terminus of the third linker peptide is linked to the N-terminus of the light chain of the antibody; or the N-terminus of the third linker peptide is linked to the C-terminus of the IL-10 monomer, and the C-terminus of the third linker peptide is linked to the N-terminus of the heavy chain of the antibody.

According to an embodiment of the present disclosure, the N-terminus of the third linker peptide is linked to the C-terminus of the VL region of the light chain, and the C-terminus of the third linker peptide is linked to the N-terminus of the IL-10 monomer; or the N-terminus of the third linker peptide is linked to the C-terminus of the IL-10 monomer, and the C-terminus of the third linker peptide is linked to the N-terminus of the CL region of the light chain; or the N-terminus of the third linker peptide is linked to the C-terminus of the VH region of the heavy chain, and the C-terminus of the third linker peptide is linked to the N-terminus of the IL-10 monomer; or the N-terminus of the third linker peptide is linked to the C-terminus of the IL-10 monomer, and the C-terminus of the third linker peptide is linked to the N-terminus of the CH1 region of the heavy chain; or the N-terminus of the third linker peptide is linked to the C-terminus of the CH2 region of the heavy chain, and the C-terminus of the third linker peptide is linked to the N-terminus of the IL-10 monomer; or the N-terminus of the third linker peptide is linked to the C-terminus of the IL-10 monomer, and the C-terminus of the third linker peptide is linked to the N-terminus of the CH3 region of the heavy chain.

According to an embodiment of the present disclosure, the N-terminus of the IL-10 monomer is linked to the C-terminus of the VL region of the light chain of the antibody by the second linker peptide, and the C-terminus of the IL-10 monomer is linked to the N-terminus of the CL region of the light chain of the antibody by the second linker peptide; or the N-terminus of the IL-10 monomer is linked to the C-terminus of the VH region of the heavy chain of the antibody by the second linker peptide, and the C-terminus of the IL-10 monomer is linked to the N-terminus of the CH1 region of the heavy chain of the antibody by the second linker peptide; or the N-terminus of the IL-10 monomer is linked to the C-terminus of the CH2 region of the heavy chain of the antibody by the second linker peptide, and the C-terminus of the IL-10 monomer is linked to the N-terminus of the CH3 region of the heavy chain of the antibody by the second linker peptide.

According to some specific implementation schemes of the present disclosure, the antibody is selected from an immune cell antibody and a tumor cell antibody.

According to some specific implementation schemes of the present disclosure, the antibody is an anti-PD-1 antibody, an anti-PD-L1 antibody, an anti-Her2 antibody, an anti-CCR8 antibody, an anti-VEGFR2 antibody, an anti-claudin18.2 antibody, an anti-CD39 antibody, an anti-SMA4D antibody, an anti-GUCY2C antibody, an anti-CTLA-4 antibody, an anti-TIM3 antibody, an anti-TIGIT antibody, an anti-CD47 antibody, or an anti-TNF α antibody.

According to some specific implementation schemes of the present disclosure, the fusion protein has amino acid sequences as shown in SEQ ID NO: 3 and SEQ ID NO: 9; or the fusion protein has amino acid sequences as shown in SEQ ID NO: 3 and SEQ ID NO: 5; or the fusion protein has amino acid sequences as shown in SEQ ID NO: 4 and SEQ ID NO: 6; or the fusion protein has amino acid sequences as shown in SEQ ID NO: 7 and SEQ ID NO: 6; or the fusion protein has amino acid sequences as shown in SEQ ID NO: 3 and SEQ ID NO: 8; or the fusion protein has amino acid sequences as shown in SEQ ID NO: 10 and SEQ ID NO: 6.

QVQLKESGPGLVQPSQTLSLTCTVSGFSLTSYNVHWVRQPPGKGLEWMGGMRYNEDTS
YNSALKSRLSISRDTSKNQVFLKMNSLQTDDTGTYYCTRDAVYGGYGGWFAYWGQGTLVTVS
SASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEAAGGPSVFLF
PPKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFSCSVMHEAL
HNHYTQKSLSLSPGK (SEQ ID NO: 3).

DTVLTQSPALAVSLGQRVTISCKASETVSSSMYSYIHWYQQKPGQQPKLLIYRASNLESGV
PARFSGSGSGTDFTLTIDPVEADDVATYFCQQSWNPWTFGGGTKLELKRTVAAPSVFIFPPSDE

QLKSGTASVVCLLNNFYPREAKVQWKVDNALQSGNSQESVTEQDSKDSTYSLSSTLTLSKADY
EKHKVYACEVTHQGLSSPVTKSFNRGECGGGGSGGGGSGGGGSGGGGSGGQGTQSENSC
THFPGNLPNMLRDLRDAFSRVKTFFQMKDQLDNLLLKESLLEDFKGYLGCQALSEMIQFYLEEV
MPQAENQDPDIKAHVNSLGENLKTLRLRLRRCHRFLPCENGGGSGGKSKAVEQVKNAFNKLQ
EKGIYKAMSEFDIFINYIEAYMTMKIRN (SEQ ID NO: 9).

DTVLTQSPALAVSLGQRVTISCKASETVSSSMYSYIHWYQQKPGQQPKLLIYRASNLESGV
PARFSGSGSGTDFTLTIDPVEADDVATYFCQQSWNPWTFGGGTKLELKRGSGSGSGSGQGTQ
SENSCTHFPGNLPNMLRDLRDAFSRVKTFFQMKDQLDNLLLKESLLEDFKGYLGCQALSEMIQ
FYLEEVMPQAENQDPDIKAHVNSLGENLKTLRLRLRRCHRFLPCENGGGSGGKSKAVEQVKN
AFNKLQEKGIYKAMSEFDIFINYIEAYMTMKIRNGSGSGSGSTVAAPSVFIFPPSDEQLKSGTASV
VCLLNNFYPREAKVQWKVDNALQSGNSQESVTEQDSKDSTYSLSSTLTLSKADYEKHKVYACE
VTHQGLSSPVTKSFNRGEC (SEQ ID NO: 5).

QVQLKESGPGLVQPSQTLSLTCTVSGFSLTSYNVHWVRQPPGKGLEWMGGMRYNEDTS
YNSALKSRLSISRDTSKNQVFLKMNSLQTDDTGTYYCTRDAVYGGYGGWFAYWGQGTLVTVS
SGSGSGSGSGQGTQSENSCTHFPGNLPNMLRDLRDAFSRVKTFFQMKDQLDNLLLKESLLED
FKGYLGCQALSEMIQFYLEEVMPQAENQDPDIKAHVNSLGENLKTLRLRLRRCHRFLPCENGG
GSGGKSKAVEQVKNAFNKLQEKGIYKAMSEFDIFINYIEAYMTMKIRNGSGSGSGSASTKGPSV
FPLAPSSKSTSGGTAALGCLVKDYFPEPVTVSWNSGALTSGVHTFPAVLQSSGLYSLSSVVTVP
SSSLGTQTYICNVNHKPSNTKVDKRVEPKSCDKTHTCPPCPAPEAAGGPSVFLFPPKPKDTLMI
SRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHYTQKSLS
LSPGK (SEQ ID NO: 4).

DTVLTQSPALAVSLGQRVTISCKASETVSSSMYSYIHWYQQKPGQQPKLLIYRASNLESGV
PARFSGSGSGTDFTLTIDPVEADDVATYFCQQSWNPWTFGGGTKLELKRTVAAPSVFIFPPSDE
QLKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO: 6).

QVQLKESGPGLVQPSQTLSLTCTVSGFSLTSYNVHWVRQPPGKGLEWMGGMRYNEDTS
YNSALKSRLSISRDTSKNQVFLKMNSLQTDDTGTYYCTRDAVYGGYGGWFAYWGQGTLVTVS
SASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEAAGGPSVFLF
PPKPKDTLMISRTPEVTCVVVDVSHEDPEVKFNWYVDGVEVHNAKTKPREEQYNSTYRVVSVL

TVLHQDWLNGKEYKCKVSNKALPAPIEKTISKAKGSGSGSGSGQGTQSENSCTHFPGNLPNM
LRDLRDAFSRVKTFFQMKDQLDNLLLKESLLEDFKGYLGCQALSEMIQFYLEEVMPQAENQDP
DIKAHVNSLGENLKTLRLRLRRCHRFLPCENGGGSGGKSKAVEQVKNAFNKLQEKGIYKAMSE
FDIFINYIEAYMTMKIRNGSGSGSGSGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSL
SLSPGK (SEQ ID NO: 7).

GQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENGGGSGGKS
KAVEQVKNAFNKLQEKGIYKAMSEFDIFINYIEAYMTMKIRNGGGGSGGGGSGGGGSGGGGS
GDTVLTQSPALAVSLGQRVTISCKASETVSSSMYSYIHWYQQKPGQQPKLLIYRASNLESGVPA
RFSGSGSGTDFTLTIDPVEADDVATYFCQQSWNPWTFGGGTKLELKRTVAAPSVFIFPPSDEQL
KSGTASVVCLLNNFYPREAKVQWKVDNALQSGNSQESVTEQDSKDSTYSLSSTLTLSKADYEK
HKVYACEVTHQGLSSPVTKSFNRGEC (SEQ ID NO: 8).

GQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENGGGSGGKS
KAVEQVKNAFNKLQEKGIYKAMSEFDIFINYIEAYMTMKIRNGGGGSGGGGSGGGGSGGGGS
GQVQLKESGPGLVQPSQTLSLTCTVSGFSLTSYNVHWVRQPPGKGLEWMGGMRYNEDTSYN
SALKSRLSISRDTSKNQVFLKMNSLQTDDTGTYYCTRDAVYGGYGGWFAYWGQGTLVTVSSA
STKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKRVEPKSCDKTHTCPPCPAPEAAGGPSVFLFPP
KPKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLVKGF
YPSDIAVEWESNGQPENNYKTTPPVLDSDGSFFLYSKLTVDKSRWQQGNVFSCSVMHEALHN
HYTQKSLSLSPGK (SEQ ID NO: 10).

In some implementation schemes, the present disclosure provides an isolated nucleic acid, and the nucleic acid encodes the fusion protein as described in the first aspect. The IL-10 molecule in the fusion protein encoded by the nucleic acid according to some specific implementation schemes of the present disclosure is in monomer form before the antibody portion binds to the target cell, it may not bind to its receptor, and may not interfere with the binding of the antibody molecule to the target cell; after the antibody binds to the target cell, a large number of IL-10 monomer molecules are aggregated, so that the two IL-10 monomers that are close to each other act as a dimer and bind to the IL-10 receptor, thereby the normal biological response is caused; and since the IL-10 monomer is difficult to trigger the biological function, the IL-10 monomer fusion protein does not produce limitations on administration doses as those of the natural dimer IL-10 molecules or its antibody fusion proteins, so that the administration dose of the IL-10 monomer fusion protein is significantly improved. The fusion protein may be used in combination with more antibody molecules, so that the targeting property and safety of the fusion protein are significantly improved, the efficacy is fully exerted, and it may treat or prevent various tumors or inflammatory diseases.

According to some specific implementation schemes of the present disclosure, the nucleic acid molecule is DNA or RNA.

In some implementation schemes, the present disclosure provides an expression vector, and the expression vector contains the nucleic acid as described in the second aspect. When the above nucleic acid molecule is linked to the vector, the nucleic acid molecule may be directly or indirectly linked to control elements on the vector, as long as these control elements may control the translation and expression and the like of the nucleic acid molecule. Certainly, these control components may come directly from the vector itself, or may be exogenous, namely they are not from the vector itself. Certainly, the nucleic acid molecule may be operably linked to the control elements. In this article, "operably linked" refers to the linkage of a foreign gene to the vector, so that the control elements inside the vector, such as a transcription control sequence and a translation control sequence, may play its expected functions in regulating the transcription and translation of the foreign gene. After the expression vector according to some specific implementation schemes of the present disclosure is introduced into an appropriate receptor cell, the expression of the fusion protein mentioned above may be effectively achieved under the mediation of a regulatory system. The IL-10 molecule in the fusion protein obtained is in monomer form before the antibody portion binds to the target cell, it may not bind to its receptor, and may not interfere with the binding of the antibody molecule to the target cell; after the antibody binds to the target cell, a large number of IL-10 monomer molecules are aggregated, so that the two IL-10 monomers that are close to each other act as a dimer and bind to the IL-10 receptor, thereby the normal biological response is caused; and since the IL-10 monomer is difficult to trigger the biological function, the IL-10 monomer fusion protein does not produce limitations on administration doses as those of the natural dimer IL-10 molecules or its antibody fusion proteins, so that the administration dose of the IL-10 monomer fusion protein is significantly improved. The fusion protein may be used in combination with more antibody molecules, so that the targeting property and safety of the fusion protein are significantly improved, the efficacy is fully exerted, and it may treat or prevent various tumors or inflammatory diseases.

According to some specific implementation schemes of the present disclosure, the expression vector is a eukaryotic expression vector.

In some implementation schemes, the present disclosure provides a recombinant cell, and the recombinant cell carries the nucleic acid as described in the second aspect, and the expression vector as described in the third aspect, or expresses the fusion protein as described in the first aspect. The recombinant cell according to the specific embodiment of the present disclosure may be used for the expression and large-scale acquisition of the fusion protein mentioned above. The IL-10 molecule in the fusion protein obtained is in monomer form before the antibody portion binds to the target cell, it may not bind to its receptor, and may not interfere with the binding of the antibody molecule to the target cell; after the antibody binds to the target cell, a large number of IL-10 monomer molecules are aggregated, so that the two IL-10 monomers that are close to each other act as a dimer and bind to the IL-10 receptor, thereby the normal biological response is caused; and since the IL-10 monomer is difficult to trigger the biological function, the IL-10 monomer fusion protein does not produce limitations on administration doses as those of the natural dimer IL-10 molecules or its antibody fusion proteins, so that the administration dose of the IL-10 monomer fusion protein is significantly improved. The fusion protein may be used in combination with more antibody molecules, so that the targeting property and safety of the fusion protein are significantly improved, the efficacy is fully exerted, and it may treat or prevent various tumors or inflammatory diseases.

According to some specific implementation schemes of the present disclosure, the recombinant cell is a mammalian cell, the mammalian is, for example, human, monkey, rabbit, dog, and cow; and the mammalian cell is, for example, the human HEK-293F cell or CHO-K1 cell.

According to some specific implementation schemes of the present disclosure, the recombinant cell does not include an animal germ cell, a fertilized egg, or an embryonic stem cell.

In some implementation schemes, the present disclosure provides a use of the fusion protein, the nucleic acid, the expression vector, or the recombinant cell mentioned above in preparation of a drug, and the drug is used to treat or prevent tumors or inflammatory diseases. According to some specific implementation schemes of the present disclosure, the drug may effectively treat or alleviate the tumors and inflammatory diseases, and has the relatively high safety.

In some implementation schemes, the present disclosure provides a pharmaceutical composition, containing the fusion protein, the nucleic acid, the expression vector, or the recombinant cell mentioned above. The pharmaceutical composition according to some specific implementation schemes of the present disclosure may accurately bind to the target cell to which the antibody contained in the pharmaceutical composition may bind. Before the antibody binds to the target cell, the IL-10 monomer molecule in the pharmaceutical composition has the relatively low binding activity with the IL-10 receptor. Therefore, the IL-10 monomer may not interfere with the targeting property of the antibody contained in the pharmaceutical composition, and after the antibody contained in the pharmaceutical combination binds to the target cell, the efficacy is fully exerted, and it may effectively treat or alleviate the tumors and inflammatory diseases, and has the relatively high safety.

According to some specific implementation schemes of the present disclosure, the pharmaceutical composition is used to treat or prevent tumors or inflammatory diseases.

According to some specific implementation schemes of the present disclosure, the fusion protein provided by the present disclosure may be incorporated into pharmaceutical compositions suitable for subject administration. Typically, these pharmaceutical compositions include the fusion protein provided by the present disclosure.

According to some specific implementation schemes of the present disclosure, the pharmaceutical composition further includes a pharmaceutically acceptable carrier, including any solvent, solid excipient, diluent, binder, disintegrant, other liquid excipient, dispersant, flavor correcting agent, suspension, surfactant, isotonic agent, thickener, emulsifier, preservative, solid binder, flow aid or lubricant or the like, suitable for a specific target dosage form. Except for the scope of incompatibility between any conventional excipients and the fusion protein of the present disclosure, such as any adverse biological effects generated or harmful interactions with any other components of the pharmaceutically acceptable composition, their uses are also within the scope of consideration of the present disclosure.

For example, the fusion protein of the present disclosure may be incorporated into pharmaceutical compositions suitable for parenteral administration (such as intravenous, subcutaneous, intraperitoneal, and intramuscular). These pharmaceutical combinations may be prepared in various forms. For example, liquid, semi-solid, and solid dosage forms, including but not limited to liquid solution (such as injection solution and infusion solution), dispersant or suspension, tablet, pill, powder, liposome, and suppository. The fusion protein may be administered by intravenous infusion or intramuscular or subcutaneous injection.

The terms "treatment" and "prevention" used in this article, as well as words derived from this, do not necessarily imply 100% or complete treatment or prevention. On the contrary, there are different degrees of the treatment or prevention, and those skilled in the art believe that the treatment or prevention has potential benefits or therapeutic effects. Moreover, the treatment or prevention provided by the present disclosure may include a disease being treated or prevented, such as treatment or prevention of one or more patients or symptoms of a cancer. In addition, for the purpose of this article, the "prevention" may cover delaying the onset of diseases or its symptoms or patients.

According to some specific implementation schemes of the present disclosure, the present disclosure provides a method for diagnosing, treating, preventing, or alleviating diseases, symptoms, or conditions related to tumors or inflammatory diseases in a subject, and it includes administering a therapeutically effective amount of the aforementioned fusion protein and/or the aforementioned pharmaceutical combination to the above subject.

The present disclosure is described below with reference to specific embodiments. It should be noted that these embodiments are only descriptive and do not limit the present disclosure in any ways.

If specific technologies or conditions are not specified in the embodiments, it is implemented according to technologies or conditions described in documents in this field or product specifications. Reagents or instruments used without specifying manufacturers are conventional products that may be obtained by market procurement.

### Embodiment 1: Construction of fusion protein

In this embodiment, the IL-10 monomer was inserted into different positions of the antibody molecule (the anti-PD-1 antibody was taken as an example in this embodiment) to obtain the fusion protein, herein specific experimental procedures for constructing the IL-10 monomer mainly referred to "Molecular Cloning Experiment Guidelines". The natural IL-10 monomer molecular sequence was shown in SEQ ID NO: 1. A peptide segment of the amino acid sequence as shown in SEQ ID NO: 21 was inserted between 116N and 117K sites of the natural IL-10 monomer molecular sequence (namely the first linker peptide, linker1), while the first 2 amino acids at the N-terminus of the natural IL-10 monomer were removed, to form the IL-10 monomer according to the embodiment of the present disclosure, and its sequence was shown in SEQ ID NO: 2; and the antibody heavy chain was shown in SEQ ID NO: 3, and the antibody light chain was shown in SEQ ID NO: 6. 
SPGQGTQSENSCTHFPGNLPNMLRDLRDAFSRVKTFFQMKDQLDNLLLKESLLEDFKGYL
GCQALSEMIQFYLEEVMPQAENQDPDIKAHVNSLGENLKTLRLRLRRCHRFLPCENKSKAVEQ
VKNAFNKLQEKGIYKAMSEFDIFINYIEAYMTMKIRN (SEQ ID NO: 1).
 
GQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENGGGSGGKS
 KAVEQVKNAFNKLQEKGIYKAMSEFDIFINYIEAYMTMKIRN (SEQ ID NO: 2). GGGSGG (SEQ ID NO: 21).

This embodiment constructed a total of 7 fusion proteins, and the specific structure of the fusion protein was shown in Fig. 3 (insertion positions of the IL-10 monomer were shown in the figure); and the linkage mode between the IL-10 monomer and the antibody molecule in the fusion protein was described as follows:
R0987: the IL-10 monomer was inserted between VL and CL (Position 1) of the antibody light chain, and linked by linker2 (GSGSGSGS), and the fusion protein obtained had an amino acid sequence as shown in SEQ ID NO: 3 (heavy chain) and SEQ ID NO: 5 (light chain), as shown in Fig. 3B;
R0988: the IL-10 monomer was inserted between VH and CH1 (Position 2) of the antibody heavy chain, and linked by linker2 (GSGSGSGS), and the fusion protein obtained had an amino acid sequence as shown in SEQ ID NO: 4 (heavy chain) and SEQ ID NO: 6 (light chain), as shown in Fig. 3C;
R0989: the IL-10 monomer was inserted between CH2 and CH3 (Position 3) of the antibody heavy chain, and linked by linker2 (GSGSGSGS), and the fusion protein obtained had an amino acid sequence as shown in SEQ ID NO: 7 (heavy chain) and SEQ ID NO: 6 (light chain), as shown in Fig. 3D;
R0990: the IL-10 monomer was linked to the N-terminus (Position 4) of the antibody light chain by linker, and linked by linker3 (GGGGSGGGGSGGGGSGGGGS), and the fusion protein obtained had an amino acid sequence as shown in SEQ ID NO: 3 (heavy chain) and SEQ ID NO: 8 (light chain), as shown in Fig. 3E;
R0991: the IL-10 monomer was linked to the C-terminus (Position 5) of the antibody light chain by linker, and linked by linker3 (GGGGSGGGGSGGGGSGGGGS), and the fusion protein obtained had an amino acid sequence as shown in SEQ ID NO: 3 (heavy chain) and SEQ ID NO: 9 (light chain), as shown in Fig. 3F;
R0992: the IL-10 monomer was linked to the N-terminus (Position 6) of the antibody heavy chain by linker, and linked by linker3 (GGGGSGGGGSGGGGSGGGGS), and the fusion protein obtained had an amino acid sequence as shown in SEQ ID NO: 10 (heavy chain) and SEQ ID NO: 6 (light chain), as shown in Fig. 3G;
R0993: the IL-10 monomer was linked to the C-terminus (Position 7) of the antibody heavy chain by linker, and linked by linker3 (GGGGSGGGGSGGGGSGGGGS), and the fusion protein obtained had an amino acid sequence as shown in SEQ ID NO: 11 (heavy chain) and SEQ ID NO: 6 (light chain), as shown in Fig. 3H; and
a structure diagram of control molecules (R0428, R0594, R0862, R0676, R0579, and R1049) in Embodiments 3-5 of the present disclosure was shown in Fig. 3I.

The second linker peptide (linker2) and the third linker peptide (linker3) were a flexible or rigid linker peptide, herein the second linker peptide had an amino acid sequence as shown in SEQ ID NO: 12, and the third linker peptide had an amino acid sequence as shown in SEQ ID NO: 22.

The amino acid sequences of the heavy chains and the light chains of the above fusion protein were shown in Table 1, herein, the sequence GSGSGSGS (SEQ ID NO: 12) labeled in bold italics was the amino acid sequence of the second linker peptide (linker2), the sequence GGGGSGGGGSGGGGSGGGGSG (SEQ ID NO: 22) labeled only in bold italics was the amino acid sequence of the third linker peptide (linker3), and the sequence GQGTQSENSCTHFPGNLPNMLRDLRDAFSRVKTFFQMKDQLDNLLLKESLLEDFKGYLGCQA LSEMIQFYLEEVMPQAENQDPDIKAHVNSLGENLKTLRLRLRRCHRFLPCENGGGSGGKSKAV EQVKNAFNKLQEKGIYKAMSEFDIFINYIEAYMTMKIRN (SEQ ID NO: 2) marked with only underline was the amino acid sequence of the modified IL-10 monomer.

**Table 1**

| Fusion protein | IL-10 monomer insertion position | Heavy chain | Light chain |
|---|---|---|---|
| R0987 | Between VL and CL of the light chain (Position 1) | SEQ ID NO: 3 | SEQ ID NO: 5 |
| | | (QVQLKESGPGLVQPS QTLSLTCTVSGFSLTSY NVHWVRQPPGKGLEW MGGMRYNEDTSYNSAL KSRLSISRDTSKNQVFL KMNSLQTDDTGTYYCT RDAVYGGYGGWFAYW GQGTLVTVSSASTKGP SVFPLAPSSKSTSGGTA ALGCLVKDYFPEPVTVS WNSGALTSGVHTFPAV LQSSGLYSLSSVVTVPS SSLGTQTYICNVNHKPS NTKVDKRVEPKSCDKT HTCPPCPAPEAAGGPS VFLFPPKPKDTLMISRT PEVTCVVVDVSHEDPE VKFNWYVDGVEVHNAK TKPREEQYNSTYRVVS VLTVLHQDWLNGKEYK CKVSNKALPAPIEKTISK AKGQPREPQVYTLPPS REEMTKNQVSLTCLVK GFYPSDIAVEWESNGQ PENNYKTTPPVLDSDG SFFLYSKLTVDKSRWQ QGNVFSCSVMHEALHN HYTQKSLSLSPGK) | (DTVLTQSPALAVSLGQRVTI SCKASETVSSSMYSYIHWY QQKPGQQPKLLIYRASNLES GVPARFSGSGSGTDFTLTID PVEADDVATYFCQQSWNP WTFGGGTKLELKRGSGSGS GSGQGTQSENSCTHFPGNL PNMLRDLRDAFSRVKTFFQ MKDQLDNLLLKESLLEDFKG YLGCQALSEMIQFYLEEVMP QAENQDPDIKAHVNSLGEN LKTLRLRLRRCHRFLPCENG GGSGGKSKAVEQVKNAFNK LQEKGIYKAMSEFDIFINYIE AYMTMKIRNGSGSGSGSTV AAPSVFIFPPSDEQLKSGTA SVVCLLNNFYPREAKVQWK VDNALQSGNSQESVTEQDS KDSTYSLSSTLTLSKADYEK HKVYACEVTHQGLSSPVTK SFNRGEC) |
| R0988 | Between VH and CH1 of the heavy chain (Position 2) | SEQ ID NO: 4 | SEQ ID NO: 6 |
| | | (QVQLKESGPGLVQPS QTLSLTCTVSGFSLTSY NVHWVRQPPGKGLEW MGGMRYNEDTSYNSAL KSRLSISRDTSKNQVFL KMNSLQTDDTGTYYCT RDAVYGGYGGWFAYW GQGTLVTVSSGSGSGS GSGQGTQSENSCTHFP GNLPNMLRDLRDAFSR VKTFFQMKDQLDNLLLK ESLLEDFKGYLGCQALS | (DTVLTQSPALAVSLGQRVTI SCKASETVSSSMYSYIHWY QQKPGQQPKLLIYRASNLES GVPARFSGSGSGTDFTLTID PVEADDVATYFCQQSWNP WTFGGGTKLELKRTVAAPS VFIFPPSDEQLKSGTASVVC LLNNFYPREAKVQWKVDNA LQSGNSQESVTEQDSKDST YSLSSTLTLSKADYEKHKVY ACEVTHQGLSSPVTKSFNR GEC) |
| | | EMIQFYLEEVMPQAEN QDPDIKAHVNSLGENLK TLRLRLRRCHRFLPCEN GGGSGGKSKAVEQVKN AFNKLQEKGIYKAMSEF DIFINYIEAYMTMKIRNG SGSGSGSASTKGPSVF PLAPSSKSTSGGTAALG CLVKDYFPEPVTVSWN SGALTSGVHTFPAVLQS SGLYSLSSVVTVPSSSL GTQTYICNVNHKPSNTK VDKRVEPKSCDKTHTC PPCPAPEAAGGPSVFLF PPKPKDTLMISRTPEVT CVVVDVSHEDPEVKFN WYVDGVEVHNAKTKPR EEQYNSTYRVVSVLTVL HQDWLNGKEYKCKVSN KALPAPIEKTISKAKGQP REPQVYTLPPSREEMT KNQVSLTCLVKGFYPSD IAVEWESNGQPENNYK TTPPVLDSDGSFFLYSK LTVDKSRWQQGNVFSC SVMHEALHNHYTQKSL SLSPGK) | |
| R0989 | Between CH2 and CH3 of the heavy chain (Position 3) | SEQ ID NO: 7 | SEQ ID NO: 6 |
| | | (QVQLKESGPGLVQPS QTLSLTCTVSGFSLTSY NVHWVRQPPGKGLEW | (DTVLTQSPALAVSLGQRVTI SCKASETVSSSMYSYIHWY QQKPGQQPKLLIYRASNLES |
| | | MGGMRYNEDTSYNSAL KSRLSISRDTSKNQVFL KMNSLQTDDTGTYYCT RDAVYGGYGGWFAYW GQGTLVTVSSASTKGP SVFPLAPSSKSTSGGTA ALGCLVKDYFPEPVTVS WNSGALTSGVHTFPAV LQSSGLYSLSSVVTVPS SSLGTQTYICNVNHKPS NTKVDKRVEPKSCDKT HTCPPCPAPEAAGGPS VFLFPPKPKDTLMISRT PEVTCVVVDVSHEDPE VKFNWYVDGVEVHNAK TKPREEQYNSTYRVVS VLTVLHQDWLNGKEYK CKVSNKALPAPIEKTISK AKGSGSGSGSGQGTQ SENSCTHFPGNLPNML RDLRDAFSRVKTFFQM KDQLDNLLLKESLLEDF KGYLGCQALSEMIQFYL EEVMPQAENQDPDIKA HVNSLGENLKTLRLRLR RCHRFLPCENGGGSGG KSKAVEQVKNAFNKLQ EKGIYKAMSEFDIFINYI EAYMTMKIRNGSGSGS GSGQPREPQVYTLPPS REEMTKNQVSLTCLVK GFYPSDIAVEWESNGQ | GVPARFSGSGSGTDFTLTID PVEADDVATYFCQQSWNP WTFGGGTKLELKRTVAAPS VFIFPPSDEQLKSGTASVVC LLNNFYPREAKVQWKVDNA LQSGNSQESVTEQDSKDST YSLSSTLTLSKADYEKHKVY ACEVTHQGLSSPVTKSFNR GEC) |
| | | PENNYKTTPPVLDSDG SFFLYSKLTVDKSRWQ QGNVFSCSVMHEALHN HYTQKSLSLSPGK) | |
| R0990 | N-terminus of the light chain (Position 4) | SEQ ID NO: 3 | SEQ ID NO: 8 |
| | | (QVQLKESGPGLVQPS QTLSLTCTVSGFSLTSY NVHWVRQPPGKGLEW MGGMRYNEDTSYNSAL KSRLSISRDTSKNQVFL KMNSLQTDDTGTYYCT RDAVYGGYGGWFAYW GQGTLVTVSSASTKGP SVFPLAPSSKSTSGGTA ALGCLVKDYFPEPVTVS WNSGALTSGVHTFPAV LQSSGLYSLSSVVTVPS SSLGTQTYICNVNHKPS NTKVDKRVEPKSCDKT HTCPPCPAPEAAGGPS VFLFPPKPKDTLMISRT PEVTCVVVDVSHEDPE VKFNWYVDGVEVHNAK TKPREEQYNSTYRVVS VLTVLHQDWLNGKEYK CKVSNKALPAPIEKTISK AKGQPREPQVYTLPPS REEMTKNQVSLTCLVK GFYPSDIAVEWESNGQ PENNYKTTPPVLDSDG SFFLYSKLTVDKSRWQ | (GQGTQSENSCTHFPGNLP NMLRDLRDAFSRVKTFFQM KDQLDNLLLKESLLEDFKGY LGCQALSEMIQFYLEEVMP QAENQDPDIKAHVNSLGEN LKTLRLRLRRCHRFLPCENG GGSGGKSKAVEQVKNAFNK LQEKGIYKAMSEFDIFINYIE AYMTMKIRNGGGGSGGGG SGGGGSGGGGSGDTVLTQ SPALAVSLGQRVTISCKASE TVSSSMYSYIHWYQQKPGQ QPKLLIYRASNLESGVPARF SGSGSGTDFTLTIDPVEADD VATYFCQQSWNPWTFGGG TKLELKRTVAAPSVFIFPPS DEQLKSGTASVVCLLNNFY PREAKVQWKVDNALQSGN SQESVTEQDSKDSTYSLSS TLTLSKADYEKHKVYACEVT HQGLSSPVTKSFNRGEC) |
| | | QGNVFSCSVMHEALHN HYTQKSLSLSPGK) | |
| R0991 | C-terminus of the light chain (Position 5) | SEQ ID NO: 3 | SEQ ID NO: 9 |
| | | (QVQLKESGPGLVQPS QTLSLTCTVSGFSLTSY NVHWVRQPPGKGLEW MGGMRYNEDTSYNSAL KSRLSISRDTSKNQVFL KMNSLQTDDTGTYYCT RDAVYGGYGGWFAYW GQGTLVTVSSASTKGP SVFPLAPSSKSTSGGTA ALGCLVKDYFPEPVTVS WNSGALTSGVHTFPAV LQSSGLYSLSSVVTVPS SSLGTQTYICNVNHKPS NTKVDKRVEPKSCDKT HTCPPCPAPEAAGGPS VFLFPPKPKDTLMISRT PEVTCVVVDVSHEDPE VKFNWYVDGVEVHNAK TKPREEQYNSTYRVVS VLTVLHQDWLNGKEYK CKVSNKALPAPIEKTISK AKGQPREPQVYTLPPS REEMTKNQVSLTCLVK GFYPSDIAVEWESNGQ PENNYKTTPPVLDSDG SFFLYSKLTVDKSRWQ QGNVFSCSVMHEALHN HYTQKSLSLSPGK) | (DTVLTQSPALAVSLGQRVTI SCKASETVSSSMYSYIHWY QQKPGQQPKLLIYRASNLES GVPARFSGSGSGTDFTLTID PVEADDVATYFCQQSWNP WTFGGGTKLELKRTVAAPS VFIFPPSDEQLKSGTASVVC LLNNFYPREAKVQWKVDNA LQSGNSQESVTEQDSKDST YSLSSTLTLSKADYEKHKVY ACEVTHQGLSSPVTKSFNR GECGGGGSGGGGSGGGG SGGGGSGGQGTQSENSCT HFPGNLPNMLRDLRDAFSR VKTFFQMKDQLDNLLLKESL LEDFKGYLGCQALSEMIQFY LEEVMPQAENQDPDIKAHV NSLGENLKTLRLRLRRCHRF LPCENGGGSGGKSKAVEQ VKNAFNKLQEKGIYKAMSEF DIFINYIEAYMTMKIRN) |
| R0992 | N-terminus of the antibody heavy chain (Position 6) | SEQ ID NO: 10 | SEQ ID NO: 6 |
| | | (GQGTQSENSCTHFPG NLPNMLRDLRDAFSRV KTFFQMKDQLDNLLKE SLLEDFKGYLGCQALSE MIQFYLEEVMPQAENQ DPDIKAHVNSLGENLKT LRLRLRRCHRFLPCEN GGGSGGKSKAVEQVKN AFNKLQEKGIYKAMSEF DIFINYIEAYMTMKIRNG GGGSGGGGSGGGGSG GGGSGQVQLKESGPGL VQPSQTLSLTCTVSGFS LTSYNVHWVRQPPGKG LEWMGGMRYNEDTSY NSALKSRLSISRDTSKN QVFLKMNSLQTDDTGT YYCTRDAVYGGYGGWF AYWGQGTLVTVSSAST KGPSVFPLAPSSKSTSG GTAALGCLVKDYFPEPV TVSWNSGALTSGVHTF PAVLQSSGLYSLSSVVT VPSSSLGTQTYICNVNH KPSNTKVDKRVEPKSC DKTHTCPPCPAPEAAG GPSVFLFPPKPKDTLMI SRTPEVTCVVVDVSHE DPEVKFNWYVDGVEVH NAKTKPREEQYNSTYR | (DTVLTQSPALAVSLGQRVTI SCKASETVSSSMYSYIHWY QQKPGQQPKLLIYRASNLES GVPARFSGSGSGTDFTLTID PVEADDVATYFCQQSWNP WTFGGGTKLELKRTVAAPS VFIFPPSDEQLKSGTASVVC LLNNFYPREAKVQWKVDNA LQSGNSQESVTEQDSKDST YSLSSTLTLSKADYEKHKVY ACEVTHQGLSSPVTKSFNR GEC) |
| | | VVSVLTVLHQDWLNGK EYKCKVSNKALPAPIEK TISKAKGQPREPQVYTL PPSREEMTKNQVSLTC LVKGFYPSDIAVEWESN GQPENNYKTTPPVLDS DGSFFLYSKLTVDKSR WQQGNVFSCSVMHEAL HNHYTQKSLSLSPGK) | |
| R0993 | C-terminus of the antibody heavy chain (Position 7) | SEQ ID NO: 11 | SEQ ID NO: 6 |
| | | (GQGTQSENSCTHFPG NLPNMLRDLRDAFSRV KTFFQMKDQLDNLLLKE SLLEDFKGYLGCQALSE MIQFYLEEVMPQAENQ DPDIKAHVNSLGENLKT LRLRLRRCHRFLPCEN GGGSGGKSKAVEQVKN AFNKLQEKGIYKAMSEF DIFINYIEAYMTMKIRNG GGGSGGGGSGGGGSG GGGSGQVQLKESGPGL VQPSQTLSLTCTVSGFS LTSYNVHWVRQPPGKG LEWMGGMRYNEDTSY NSALKSRLSISRDTSKN QVFLKMNSLQTDDTGT YYCTRDAVYGGYGGWF AYWGQGTLVTVSSAST KGPSVFPLAPSSKSTSG GTAALGCLVKDYFPEPV | (DTVLTQSPALAVSLGQRVTI SCKASETVSSSMYSYIHWY QQKPGQQPKLLIYRASNLES GVPARFSGSGSGTDFTLTID PVEADDVATYFCQQSWNP WTFGGGTKLELKRTVAAPS VFIFPPSDEQLKSGTASVVC LLNNFYPREAKVQWKVDNA LQSGNSQESVTEQDSKDST YSLSSTLTLSKADYEKHKVY ACEVTHQGLSSPVTKSFNR GEC) |
| | | TVSWNSGALTSGVHTF PAVLQSSGLYSLSSVVT VPSSSLGTQTYICNVNH KPSNTKVDKRVEPKSC DKTHTCPPCPAPEAAG GPSVFLFPPKPKDTLMI SRTPEVTCVVVDVSHE DPEVKFNWYVDGVEVH NAKTKPREEQYNSTYR VVSVLTVLHQDWLNGK EYKCKVSNKALPAPIEK TISKAKGQPREPQVYTL PPSREEMTKNQVSLTC LVKGFYPSDIAVEWESN GQPENNYKTTPPVLDS DGSFFLYSKLTVDKSR WQQGNVFSCSVMHEAL HNHYTQKSLSLSPGK) | |

### Embodiment 2: Preparation of fusion protein

A plasmid containing a target gene (encoding the fusion protein in Embodiment 1) was constructed and prepared with a conventional method. After the plasmid containing the target gene formed a cationic complex with a transfection reagent PEI, it was introduced into a host cell Expi293. During the intracellular period of the plasmid, a foreign gene on the plasmid underwent transcriptional translation in the cell, thereby the fusion protein was obtained, and specific experimental procedures were as follows:
Expi293 was cultured at 37°C, 8% carbon dioxide, and 130 rpm, and 2E6 cells were inoculated into a 1 L shake flask by cell counting before transfection. The culture system was about 300 mL. A transfection complex was prepared for the transfection: firstly, 750 µg of the target plasmid was added to a 50 mL centrifuge tube containing a 15 mL Opti-MEM reagent, gently mixed uniformly, and labeled as a tube A; 1.5 mg of the transfection reagent PEI was added to a 50 mL centrifuge tube containing a 15 mL Opti-MEM reagent, gently mixed uniformly, incubated at a room temperature for 5 min, and labeled as a tube B; the PEI diluent in the tube B was dropwise added to the DNA diluent in the tube A, it was incubated at the room temperature for 15 min after being gently mixed uniformly, and after the incubation, a PEl-target plasmid complex was added to the Expi293 cells, and continuously cultured in a shaker at 37 °C until D5-D10, and a sample was collected.

Transient cell expression solution was centrifuged at 9000 rpm/20 min, and a supernatant was collected and then subjected to 0.22 µM filter membrane sterilization and filtration. Purification was performed with a ProA affinity chromatography. The process was as follows: an AKTA avant 150 chromatography device was used, at least 5CV equilibrium buffer solution (10 mM PBS) was used to equilibrate a chromatography column (such as MabSelectSuRe LX, GE), the sample was loaded onto the chromatography column, so that a target protein was adsorbed on the chromatography column while other impurities penetrated and separated. After completing the sample loading, the chromatography column was rinsed again with at least 5CV equilibrium buffer solution (10 mM PBS), and then the target protein was eluted with elution buffer solution (20 mM NaAc, pH=3.4). Neutralization buffer solution (1 M Tris, pH=8.0) was added to a collection tube in advance, and the volume of the neutralization buffer solution added was determined according to the estimated content of the sample eluted. Generally, 10% of the elution volume was added.

Expression data of the IL-10 monomer fusion protein was shown in Table 2. Except for R0987 with an expression level of 97.731 mg/mL, the transient expression levels of all other fusion proteins were greater than 100 mg/L, and the expression level of R0990 even reached 194.409 mg/L. After one-step affinity purification, the sample was detected by SEC-HPLC, and the target purity was above 85%. The purity of R0989 was even greater than 95%. The sample was detected by SDS-PAGE and showed the correct distribution of the light and heavy chains, and electrophoresis results were shown in Fig. 4. It might be concluded that the IL-10 monomer fusion proteins at the different insertion positions all showed the good production properties.

**Table 2**

| Sample | Protein content (mg) | Titer (mg/L) | SEC-Purity after one-step purification | | |
|---|---|---|---|---|---|
| | | | Aggregate (%) | Target peak (%) | Fragment (%) |
| R0987 | 26.076 | 97.731 | 12.39 | 85.56 | 2.05 |
| R0988 | 29.574 | 111.632 | 12.78 | 85.35 | 1.87 |
| R0989 | 37.921 | 141.098 | 3.07 | 95.77 | 1.16 |
| R0990 | 52.003 | 194.409 | 5.38 | 90.77 | 3.85 |
| R0991 | 45.921 | 170.571 | 4.89 | 94.94 | 0.17 |
| R0992 | 52.958 | 191.448 | 6.14 | 89.25 | 4.61 |
| R0993 | 33.037 | 124.242 | 5.32 | 93.45 | 1.23 |

### Embodiment 3: In vitro activity of fusion protein

### 3.1. Detection of binding activity of antibody terminus and IL-10 monomer terminus in fusion protein by flow cytometry fluorescence sorting technology (FACS)

All fusion protein molecules, R0428 (a positive control antibody without IL-10, its heavy chain amino acid sequence and light chain amino acid sequence were shown in SEQ ID NOs: 13 and 14), or R0594 (an IL-10-Fc fusion protein control, its amino acid sequence was shown in SEQ ID NO: 15, herein IL-10 removed the first 2 amino acids of the natural IL-10 and did not insert the first linker peptide) protein molecules were diluted to an initial concentration of 400 nM and a volume of 180 µl, and it was diluted in a 3-fold gradient (60 µl sample + 120 µl dilution Buffer), to obtain a total of 11 concentration gradient points. CHO-mPD1 or CHO-hIL-10R cells were centrifuged at 250 g for 5 min, a supernatant was discarded, the cell density was adjusted to 2E+06 by a PBS buffer with 3% BSA, and it was evenly divided into a 96-well V-shaped plate according to 100 µl/tube; the fusion protein molecules diluted above were added to cells, and incubated at 100 µL/well and 2-8°C for 0.5 h; the 96-well plate was taken out, and centrifuged at 250 g for 5 min, and a supernatant was removed carefully; the PBS buffer with 3% BSA was used to prepare a PE fluorescent secondary antibody (1:500 dilution), it was added to the corresponding 96-well plate according to the volume of 100 µL/well, and the cells were resuspended, and incubated at 2-8°C for 30 min; the 96-well plate was taken out, and centrifuged at 250 g for 5 min, a supernatant was removed carefully, the PBS buffer with 3% BSA was added at 200 µL/well, and it was centrifuged again at 250 g for 5 min, and a supernatant was removed carefully; and it was resuspended with 1xPBS according to the volume of 100 µL/well, and FACS detection was performed after the resuspension.
Heavy chain sequence of R0428: 
QVQLKESGPGLVQPSQTLSLTCTVSGFSLTSYNVHWVRQPPGKGLEWMGGMRYNEDTS
YNSALKSRLSISRDTSKNQVFLKMNSLQTDDTGTYYCTRDAVYGGYGGWFAYWGQGTLVTVS
SAKTTPPSVYPLAPGSAAQTNSMVTLGCLVKGYFPEPVTVTWNSGSLSSGVHTFPAVLQSDLY
 
TLSSSVTVPSSTWPSQTVTCNVAHPASSTKVDKKIVPRDCGCKPCICTVPEVSSVFIFPPKPKD
VLTITLTPKVTCVVVDISKDDPEVQFSWFVDDVEVHTAQTKPREEQFNSTFRSVSELPIMHQDW
LNGKEFKCRVNSAAFPAPIEKTISKTKGRPKAPQVYTIPPPKEQMAKDKVSLTCMITNFFPEDITV
EWQWNGQPAENYKNTQPIMDTDGSYFVYSKLNVQKSNWEAGNTFTCSVLHEGLHNHHTEKS
LSHSPGK (SEQ ID NO: 13).
Light chain sequence of R0428: 
DTVLTQSPALAVSLGQRVTISCKASETVSSSMYSYIHWYQQKPGQQPKLLIYRASNLESGV
PARFSGSGSGTDFTLTIDPVEADDVATYFCQQSWNPWTFGGGTKLELKRADAAPTVSIFPPSS
EQLTSGGASVVCFLNNFYPKDINVKWKIDGSERQNGVLNSWTDQDSKDSTYSMSSTLTLTKDE
YERHNSYTCEATHKTSTSPIVKSFNRNEC (SEQ ID NO: 14).
R0594 contained two identical peptide chains, and the amino acid sequence of one of which was as follows:

GQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENKSKAVEQV
KNAFNKLQEKGIYKAMSEFDIFINYIEAYMTMKIRNGGGSGSGSGSEPKSSDKTHTCPPCPAPE
LAGAPSVFLFPPKPKDTLMISRTPEVTCVVVDVSHEDPEVKFNWYVDGVEVHNAKTKPREEQY
NSTYRVVSVLTVLHQDWLNGKEYKCKVSNKQLPAPIEKTISKAKGQPREPQVYTLPPSRDELTK
NQVSLTCLVKGFYPSDIAVEWESNGQPENNYKTTPPVLDSDGSFFLYSKLTVDKSRWQQGNVF
SCSVMHEALHNHYTQKSLSLSPGK (SEQ ID NO: 15).

The antibody terminus binding activity detection results were shown in Fig. 5. The fusion proteins R0987, R0988, R0989, and R0991 had the stronger antibody terminus binding activity, while R0990 is the weakest. It was indicated that the insertion of the IL-10 monomer between the light chains VL and CL, or between the heavy chains VH and CH1, or between the heavy chains CH2 and CH3 did not affect the antibody binding activity at the C-terminus of the light chain.

The binding activity detection results of the IL-10 monomer terminus to IL-10Rα were shown in Fig. 6, herein the binding activity of the IL-10 monomer terminus to IL-10Rα in the fusion proteins R0989 and R0993 was significantly weak, which might be related to the fact that the insertion position of the IL-10 monomer was close to the C-terminus of the antibody, while the binding activity of other fusion proteins to IL-10Rα was relatively close, and consistent with the positive control molecule R0594. It was indicated that the IL-10 monomer inserted into the antibody domain might still form the expected spatial structure after recombinant expression and might bind to IL-10Rα normally.

### 3.2. Detection of activity of IL-10 monomer terminus of IL-10 monomer fusion protein by reporter gene method

All fusion protein molecules and control protein molecules R0579 (an IL-10 positive antibody fusion protein control, its heavy chain amino acid sequence was shown in SEQ ID NO: 23, and its light chain amino acid sequence was shown in SEQ ID NO: 14, herein IL-10 removed the first 2 amino acids of the natural IL-10 and did not insert the first linker peptide) were diluted to an initial concentration of 20 µg/mL and a volume of 450 µl with 1640+10% FBS, and it was diluted in a 3-fold gradient (150 µl + 300 µl), to obtain a total of 9 concentrations.

Cell preparation: HEK293-hlL-10-Report gene cells (Cat#GM-C07927, Genomeditech) that were currently cultured were taken out and observed under a microscope. The cells were normal adherence and transparent in particles, and the cells in moderate density might be used as effector cells for the experiment; the above cells were digested with Tris-EDTA (TE), after the digestion was stopped, it was centrifuged at 300 g for 4 min to remove a supernatant, resuspend with 1% FBS-PBS, and washed again; and a supernatant was discarded, and finally a culture medium was used to resuspend the cells, after the cells were counted, the cell density was adjusted to 5×10⁵ cells/mL. According to the experimental design above, the diluted drug was firstly added to a corresponding well plate according to a volume of 50 µL/well, and then the above resuspended cells were also added according to the volume of 50 µL/well, 100 µL of a medium was supplemented to wells of Medium only, 50 µL of a medium was supplemented to wells of Cell only, and finally the final volumes of all wells were 100 µL. The 96-well plate was continuously cultured in an incubator for 16 h. A Bright-LumiTM firefly luciferase detection reagent was thawed in advance and equilibrated to a room temperature. After 16 h of the cell culture, the cell culture plate was taken out and equilibrated to the room temperature for 10 min (not more than 30 min). 100 µL of the Bright-LumiTM firefly luciferase detection reagent was added to each well, incubated at the room temperature for 5-10 min. A signal was detected by using a chemiluminescence mode in a multifunctional microplate reader.

The detection results were shown in Fig. 7, and the reporter gene results were similar to the Binding results. The binding activity of the IL-10 monomer terminus of the fusion proteins R0989 and R0993 was relatively low, while the activity of the positive control R0579 was much stronger than that of the fusion protein in this detection. The detection results of the reporter gene method indicated that the binding activity of the modified IL-10 monomer was lower than that of the IL-10 molecule in dimer form.

### Embodiment 4: Effect of fusion protein on MC38 tumor model mouse

The inventor conducted an in vivo experiment on mice based on the detection in Embodiment 3, and the specific experimental procedures were as follows:
Cell culture: MC38 tumor cells (source: National Experimental Cell Resource Sharing Platform, resource number: 3111C0001CCC000523) were cultured in DMEM (supplier: Gibco, product number: 11965084) with the addition of 10% FBS (inactivated fetal bovine serum, supplier: Gibco, product number: 10091148), 100 U/mL penicillin, and 100% FBS µg/mL streptomycin. The temperature of an incubator was controlled at 37 °C and 5% carbon dioxide was supplemented. The tumor cells were passaged twice a week and digested with a pancreatic enzyme EDTA.

Inoculation: the MC38 tumor cells in the logarithmic growth phase were collected, and centrifuged at 250 g for 10 min after being digested. It was washed twice with cold PBS and cell counting was performed, and PBS was adjusted to a cell concentration of 3×10⁶ cells/mL. The right abdomens of the mice were shaved in advance for skin preparation, each mouse was inoculated with 0.1 mL of cell suspension, and the inoculation amount was 3×10⁵ tumor cells per mouse.

Grouping: On the 6th-8th day after the inoculation, all animal tumors were measured and weighed. In order to avoid the impact of the initial tumor volume on treatment effectiveness, the mice were randomly assigned to groups according to the tumor volumes, and it was ensured that the average tumor volume of all groups was comparable, so that the treatment effect was comparable at the baseline.

Observation: After the inoculation, the incidence rate and the mortality rate of the animals were checked every day. After the start of treatment, the fusion proteins R0987, R0988, R0989, R0991, R0992, R0993, and R0862 (namely Isotype, a negative control antibody; its heavy chain amino acid sequence and light chain amino acid sequence were shown in SEQ ID NOs: 16 and 17), R0428, R0676 (an IL-10-Fc fusion protein control, its amino acid sequence was shown in SEQ ID NO: 18, herein IL-10 removed the first 2 amino acids of the natural IL-10 and did not insert the first linker peptide), and R0579 protein molecules were intraperitoneally injected into the mice at doses of 0.61, 0.61, 0.61, 0.61, 0.61, 0.61, 0.5, 0.5, 0.3, and 0.6 mg/kg respectively, and Q3D×3 (administered once every 3 days, a total of 3 times), the tumor volume was measured for 3 times every week, the effects of animal tumor growth and treatment on normal behaviors were checked and recorded, such as mobility, food and water consumption, weight gain/loss, eyes/hairs/skins, and other effects.
Heavy chain sequence of R0862: QVQLVESGGGVVQPGRSLRLSCAASGFTFSSYGMHWVRQAPGKGLEWVAAIWYDGSNK 
YYTDSVKGRFTISRDNSKNTLYLQMNSLRAEDTAVYYCARDLRGVMYFDLWGRGTLVTVSSAS
TKGPSVFPLAPSSKSTSGGTAALGCLVKDYFPEPVTVSWNSGALTSGVHTFPAVLQSSGLYSLS
SVVTVPSSSLGTQTYICNVNHKPSNTKVDKRVEPKSCDKTHTCPPCPAPEAAGGPSVFLFPPK
PKDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREEMTKNQVSLTCLVKGF
YPSDIAVEWESNGQPENNYKTTPPVLDSDGSFFLYSKLTVDKSRWQQGNVFSCSVMHEALHN
HYTQKSLSLSPGK (SEQ ID NO: 16).
Light chain sequence of R0862: 
EIVLTQSPATLSLSPGERATLSCRASQSVSRYLAWYQQKPGQAPRLLIYDASNRATGIPARF
SGSGSGTDFTLTISSLEPEDFAVYYCQLRNNWPLTFGGGTKVEIKRTVAAPSVFIFPPSDEQLKS
GTASVVCLLNNFYPREAKVQWKVDNALQSGNSQESVTEQDSKDSTYSLSSTLTLSKADYEKHK
VYACEVTHQGLSSPVTKSFNRGEC (SEQ ID NO: 17).
R0676 contained two identical peptide chains, and the amino acid sequence of one of which was as follows: 
DKTHTCPPCPAPEAEGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGKGGGGSGGGGSGGGGSGGG
GSSPGQGTQSENSCTHFPGNLPNMLRDLRDAFSRVKTFFQMKDQLDNLLLKESLLEDFKGYL
GCQALSEMIQFYLEEVMPQAENQDPDIKAHVNSLGENLKTLRLRLRRCHRFLPCENKSKAVEQ
VKNAFNKLQEKGIYKAMSEFDIFINYIEAYMTMKIRN (SEQ ID NO: 18).
Heavy chain sequence of R0579: 
QVQLKESGPGLVQPSQTLSLTCTVSGFSLTSYNVHWVRQPPGKGLEWMGGMRYNEDTS
YNSALKSRLSISRDTSKNQVFLKMNSLQTDDTGTYYCTRDAVYGGYGGWFAYWGQGTLVTVS
SAKTTPPSVYPLAPGSAAQTNSMVTLGCLVKGYFPEPVTVTWNSGSLSSGVHTFPAVLQSDLY
TLSSSVTVPSSTWPSQTVTCNVAHPASSTKVDKKIVPRDCGCKPCICTVPEVSSVFIFPPKPKD
VLTITLTPKVTCVVVAISKDDPEVQFSWFVDDVEVHTAQTKPREEQFNSTFRSVSELPIMHQDW
LNGKEFKCRVNSAAFPAPIEKTISKTKGRPKAPQVYTIPPPKEQMAKDKVSLTCMITNFFPEDITV
EWQWNGQPAENYKNTQPIMDTDGSYFVYSKLNVQKSNWEAGNTFTCSVLHEGLHNHHTEKS
LSHSPGAGGGGSGGGGSGGGGSGGGGSGSPGQGTQSENSCTHFPGNLPNMLRDLRDAFS
 
RVKTFFQMKDQLDNLLLKESLLEDFKGYLGCQALSEMIQFYLEEVMPQAENQDPDIKAHVNSL
GENLKTLRLRLRRCHRFLPCENKSKAVEQVKNAFNKLQEKGIYKAMSEFDIFINYIEAYMTMKIR
N (SEQ ID NO: 23).
Light chain sequence of R0579:

DTVLTQSPALAVSLGQRVTISCKASETVSSSMYSYIHWYQQKPGQQPKLLIYRASNLESGV
PARFSGSGSGTDFTLTIDPVEADDVATYFCQQSWNPWTFGGGTKLELKRADAAPTVSIFPPSS
EQLTSGGASVVCFLNNFYPKDINVKWKIDGSERQNGVLNSWTDQDSKDSTYSMSSTLTLTKDE
YERHNSYTCEATHKTSTSPIVKSFNRNEC (SEQ ID NO: 14).

Endpoints: the primary endpoint of the experiment was whether the tumor growth was delayed or whether the mice were cured. Tumor Growth Inhibition (TGI) was calculated according to the volume of each group of tumors. Calculation method: Independent sample T-test might be used for comparison between two groups. One-Way ANOVA was used for comparison of more than 3 groups. If the F-value showed a significant difference, post hoc analysis between multiple groups might be conducted. Data was processed by using GraphPad Prism, and when p<0.05, it indicated the significant statistical difference. Tumor volume V=0.5a×b2, a and b represented long and short diameters of the tumor respectively. TGI(%) = [1-(Ti-T0)/(Vi-V0)]×100, Ti was an average tumor volume of a treatment group on day i, T0 was an average tumor volume of the treatment group at the beginning of treatment, Vi was an average tumor volume of a solvent control group on day i, and V0 was an average tumor volume of the solvent control group S at the beginning of treatment. TGI was used to represent the effects of the drug on the tumor growth inhibition.

The experimental results were shown in Fig. 8. After the treatment with the fusion proteins R0987, R0991, and R0993, the tumor growth rate in the mice was decreased and the tumor volume was smaller; and compared to monoclonal antibodies or IL-10 fusion proteins, the fusion proteins R0987, R0991 and R0993 had the better anti-tumor effects.

### Embodiment 5: Effect of fusion protein on CT26 tumor model mouse

The inventor further verified the fusion protein on the basis of the experimental results obtained in Embodiment 4, and the specific experimental procedures were as follows:
Cell culture: CT26 tumor cells (supplier: Cell Bank of Chinese Academy of Sciences, product number: NA) were cultured in RPMI-1640 (supplier: Gibco, product number: 11875085) with the addition of 10% FBS (inactivated fetal bovine serum, supplier: Gibco, product number: 10091148), 100 U/mL penicillin, and 100 µg/mL streptomycin. The temperature of an incubator was controlled at 37°C and 5% carbon dioxide was supplemented. The tumor cells were passaged twice a week and digested with a pancreatic enzyme EDTA.

Inoculation: the cells in the logarithmic growth phase were collected, and centrifuged at 250 g for 10 min after being digested. It was washed twice with cold PBS and cell counting was performed, and the cell concentration in PBS was adjusted to 1×10⁶/mL. The right abdomens of the mice were shaved in advance for skin preparation, each mouse was inoculated with 0.1 mL of cell suspension, and the inoculation amount was 1×10⁵ tumor cells per mouse.

Grouping: On the 6th-8th day after the inoculation, all animal tumors were measured and weighed. In order to avoid the impact of the initial tumor volume on treatment effectiveness, the mice were randomly assigned to groups according to the tumor volumes, and it was ensured that the average tumor volume of all groups was comparable, so that the treatment effect was comparable at the baseline.

Observation: the use and care of the animals involved in this research scheme were reviewed and approved by the Animal Ethics Committee of Guangdong Fapon Biopharma Inc. before implementation. During the research period, the animals were cared and used in accordance with the SPF animal room standard operating procedures. After the inoculation, the incidence rate and the mortality rate of the animals were checked every day. After the start of treatment, the fusion proteins R0987, R0991, and R0993, as well as R0862, R0579, R0676, and R1049 (a monoclonal antibody control of anti-PD-1, its heavy chain amino acid sequence was shown in SEQ ID NO: 19, and the light chain amino acid sequence was shown in SEQ ID NO: 20) protein molecules were intraperitoneally injected into the mice at doses of 10, 10, 10, 10, 10, 5, and 7.9 mg/kg respectively, and Q3D×3 (administered once every 3 days, a total of 3 times), the tumor volume was measured for twice every week, the effects of animal tumor growth and treatment on normal behaviors were checked and recorded, such as mobility, food and water consumption, weight gain/loss, eyes/hairs/skins, and other effects.
R1049 heavy chain sequence: 
QVQLKESGPGLVQPSQTLSLTCTVSGFSLTSYNVHWVRQPPGKGLEWMGGMRYNEDTS
YNSALKSRLSISRDTSKNQVFLKMNSLQTDDTGTYYCTRDAVYGGYGGWFAYWGQGTLVTVS
SASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEAAGGPSVFLF
PPKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFSCSVMHEAL
 HNHYTQKSLSLSPGK (SEQ ID NO: 19).
R1049 light chain sequence:

DTVLTQSPALAVSLGQRVTISCKASETVSSSMYSYIHWYQQKPGQQPKLLIYRASNLESGV
PARFSGSGSGTDFTLTIDPVEADDVATYFCQQSWNPWTFGGGTKLELKRTVAAPSVFIFPPSDE
QLKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO: 20).

Endpoints: the primary endpoint of the experiment was whether the tumor growth was delayed or whether the mice were cured. TGI was calculated according to the volume of each group of tumors. Calculation method: Independent sample T-test might be used for comparison between two groups. One-Way ANOVA was used for comparison of more than 3 groups. If the F-value showed a significant difference, post hoc analysis between multiple groups might be conducted. Data was processed by using GraphPad Prism, and when p<0.05, it indicated the significant statistical difference. Tumor volume V=0.5a×b2, a and b represented long and short diameters of the tumor respectively. TGI(%)=[1-(Ti-T0)/(Vi-V0)]×100, Ti was an average tumor volume of a treatment group on day i, T0 was an average tumor volume of the treatment group at the beginning of treatment, Vi was an average tumor volume of a solvent control group on day i, and V0 was an average tumor volume of the solvent control group S at the beginning of treatment. TGI was used to represent the effects of the drug on the tumor growth inhibition. According to the specific experiment, the survival curve was also one of the standards for measuring the drug efficacy.

The experimental results, as shown in Fig. 9, showed that compared to the fusion proteins R0987 and R0993, the tumor growth rate in the mice was significantly decreased after treatment with the fusion protein R0991, and the tumor volume was smaller, it indicated the better anti-tumor effect.

In this description, the descriptions of the reference terms such as "one embodiment", "some embodiments", "examples", "specific examples", or "some examples" mean that specific features, structures, materials, or features described in combination with the embodiments or examples are contained in at least one embodiment or example of the present disclosure. In this description, the schematic expressions of the above terms do not necessarily refer to the same embodiments or examples. Moreover, the specific features, structures, materials, or features described may be combined in an appropriate manner in any one or more embodiments or examples. In addition, those skilled in the art may incorporate and combine the different embodiments or examples described in this description, as well as the features of the different embodiments or examples, without conflicting with each other.

Although the embodiments of the present disclosure are already shown and described above, it may be understood that the above embodiments are exemplary and may be understood as limitations on the present disclosure. Those of ordinary skill in the art may make changes, modifications, replacements, and variations to the above embodiments within the scope of the present disclosure.

## Claims

1. A fusion protein, comprising an antibody and an IL-10 monomer, wherein the antibody comprises two identical light chains and two identical heavy chains, one IL-10 monomer is linked to the heavy chain or the light chain of the antibody, and an N-terminus of the IL-10 monomer is not linked to a C-terminus of the heavy chain of the antibody.

2. The fusion protein according to claim 1, wherein each light chain of the antibody comprises a VL region and a CL region, and each heavy chain of the antibody comprises a VH region, a CH1 region, a CH2 region, and a CH3 region; and each light chain or each heavy chain of the antibody is linked to one IL-10 monomer;
optionally, the N-terminus of the IL-10 monomer is linked to a C-terminus of the light chain of the antibody; or
a C-terminus of the IL-10 monomer is linked to a N-terminus of the light chain of the antibody; or
the N-terminus of the IL-10 monomer is linked to a C-terminus of the VL region of the light chain of the antibody, and the C-terminus of the IL-10 monomer is linked to a N-terminus of the CL region of the light chain of the antibody; or
the C-terminus of the IL-10 monomer is linked to a N-terminus of the heavy chain of the antibody; or
the N-terminus of the IL-10 monomer is linked to a C-terminus of the VH region of the heavy chain of the antibody, and the C-terminus of the IL-10 monomer is linked to a N-terminus of the CH1 region of the heavy chain of the antibody; or
the N-terminus of the IL-10 monomer is linked to a C-terminus of the CH2 region of the heavy chain of the antibody, and the C-terminus of the IL-10 monomer is linked to a N-terminus of the CH3 region of the heavy chain of the antibody.

3. The fusion protein according to any one of claims 1-2, wherein the IL-10 monomer comprises a natural IL-10 monomer.

4. The fusion protein according to any one of claims 1-3, wherein the IL-10 monomer comprises a modified natural IL-10 monomer, wherein the modification comprises inserting a first linker peptide between the 116-th amino acid and the 117-th amino acid, and the first linker peptide is a flexible linker peptide;
optionally, the first linker peptide has an amino acid sequence as shown in SEQ ID NO: 21;
and
optionally, the IL-10 monomer has an amino acid sequence as shown in SEQ ID NO: 2.

5. The fusion protein according to any one of claims 1-4, wherein the IL-10 monomer is linked to the heavy chain or the light chain of the antibody by a second linker peptide and/or a third linker peptide; and
optionally, the second linker peptide has an amino acid sequence as shown in SEQ ID NO: 12, and the third linker peptide has an amino acid sequence as shown in SEQ ID NO: 22.

6. The fusion protein according to claim 5, wherein the IL-10 monomer is linked to a heavy chain terminus or a light chain terminus of the antibody by the second linker peptide; optionally,
a N-terminus of the second linker peptide is linked to the C-terminus of the light chain of the antibody, and a C-terminus of the second linker peptide is linked to the N-terminus of the IL-10 monomer; or
the N-terminus of the second linker peptide is linked to the C-terminus of the IL-10 monomer, and the C-terminus of the second linker peptide is linked to the N-terminus of the light chain of the antibody; or
the N-terminus of the second linker peptide is linked to the C-terminus of the IL-10 monomer, and the C-terminus of the second linker peptide is linked to the N-terminus of the heavy chain of the antibody.

7. The fusion protein according to claim 5, wherein the IL-10 monomer is linked to the heavy chain terminus or the light chain terminus of the antibody by the third linker peptide;
a N-terminus of the third linker peptide is linked to the C-terminus of the light chain of the antibody, and a C-terminus of the third linker peptide is linked to the N-terminus of the IL-10 monomer; or
the N-terminus of the third linker peptide is linked to the C-terminus of the IL-10 monomer, and the C-terminus of the third linker peptide is linked to the N-terminus of the light chain of the antibody; or
the N-terminus of the third linker peptide is linked to the C-terminus of the IL-10 monomer, and the C-terminus of the third linker peptide is linked to the N-terminus of the heavy chain of the antibody.

8. The fusion protein according to claim 5, wherein the N-terminus of the third linker peptide is linked to the C-terminus of the VL region of the light chain, and the C-terminus of the third linker peptide is linked to the N-terminus of the IL-10 monomer; or
the N-terminus of the third linker peptide is linked to the C-terminus of the IL-10 monomer, and the C-terminus of the third linker peptide is linked to the N-terminus of the CL region of the light chain; or
the N-terminus of the third linker peptide is linked to the C-terminus of the VH region of the heavy chain, and the C-terminus of the third linker peptide is linked to the N-terminus of the IL-10 monomer; or
the N-terminus of the third linker peptide is linked to the C-terminus of the IL-10 monomer, and the C-terminus of the third linker peptide is linked to the N-terminus of the CH1 region of the heavy chain; or
the N-terminus of the third linker peptide is linked to the C-terminus of the CH2 region of the heavy chain, and the C-terminus of the third linker peptide is linked to the N-terminus of the IL-10 monomer; or
the N-terminus of the third linker peptide is linked to the C-terminus of the IL-10 monomer, and the C-terminus of the third linker peptide is linked to the N-terminus of the CH3 region of the heavy chain.

9. The fusion protein according to claim 5, wherein the N-terminus of the IL-10 monomer is linked to the C-terminus of the VL region of the light chain of the antibody by the second linker peptide, and the C-terminus of the IL-10 monomer is linked to the N-terminus of the CL region of the light chain of the antibody by the second linker peptide; or
the N-terminus of the IL-10 monomer is linked to the C-terminus of the VH region of the heavy chain of the antibody by the second linker peptide, and the C-terminus of the IL-10 monomer is linked to the N-terminus of the CH1 region of the heavy chain of the antibody by the second linker peptide; or
the N-terminus of the IL-10 monomer is linked to the C-terminus of the CH2 region of the heavy chain of the antibody by the second linker peptide, and the C-terminus of the IL-10 monomer is linked to the N-terminus of the CH3 region of the heavy chain of the antibody by the second linker peptide.

10. The fusion protein according to any one of claims 1-9, wherein the antibody is selected from an immune cell antibody and a tumor cell antibody;
optionally, the antibody is an anti-PD-1 antibody, an anti-PD-L1 antibody, an anti-Her2 antibody, an anti-CCR8 antibody, an anti-VEGFR2 antibody, an anti-claudin18.2 antibody, an anti-CD39 antibody, an anti-SMA4D antibody, an anti-GUCY2C antibody, an anti-CTLA-4 antibody, an anti-TIM3 antibody, an anti-TIGIT antibody, an anti-CD47 antibody, or an anti-TNF α antibody; and
optionally, the antibody is the anti-PD-1 antibody.

11. The fusion protein according to any one of claims 1-10, wherein the fusion protein has amino acid sequences as shown in SEQ ID NO: 3 and SEQ ID NO: 9; or
the fusion protein has amino acid sequences as shown in SEQ ID NO: 3 and SEQ ID NO: 5; or
the fusion protein has amino acid sequences as shown in SEQ ID NO: 4 and SEQ ID NO: 6; or
the fusion protein has amino acid sequences as shown in SEQ ID NO: 7 and SEQ ID NO: 6; or
the fusion protein has amino acid sequences as shown in SEQ ID NO: 3 and SEQ ID NO: 8; or
the fusion protein has amino acid sequences as shown in SEQ ID NO: 10 and SEQ ID NO: 6.

12. A nucleic acid, wherein the nucleic acid encodes the fusion protein according to any one of claims 1-11.

13. An expression vector, wherein the expression vector contains the nucleic acid according to claim 12.

14. A recombinant cell, wherein the recombinant cell carries the nucleic acid according to claim 12, or the expression vector according to claim 13 or expresses the fusion protein according to any one of claims 1-11.

15. Use of the fusion protein according to any one of claims 1-11, the nucleic acid according to claim 12, the expression vector according to in claim 13, or the recombinant cell according to claim 14 in preparation of a drug for treating or preventing tumors or inflammatory diseases.

16. A pharmaceutical composition comprising the fusion protein according to any one of claims 1-11, the nucleic acid according to claim 12, the expression vector according to claim 13, or the recombinant cell according to claim 14.

17. The pharmaceutical composition according to claim 16, wherein the pharmaceutical composition is used to treat or prevent the tumors or inflammatory diseases.
